# EUROPEAN PATENT APPLICATION

(11) **EP 4 410 972 A1**
(43) Date of publication of application: **07.08.2024**
(21) Application number: 23305151.5
(22) Date of filing: 03.02.2023
(51) Int. Cl.: C12N 9/10

(54) **MODIFIED BENZALACETONE SYNTHASE ENZYMES AND USES THEREOF**

(71) Applicant: BGene Genetics, 38000 Grenoble (FR)
(72) Inventor: GOUSSE, Matthieu, 38000 GRENOBLE (FR); DE ANDRADE, Ricardo, 38000 GRENOBLE (FR); CHANDOR-PROUST, Alexia, 38000 GRENOBLE (FR)
(74) Representative: Plasseraud IP

(57) **Abstract**

The present invention is in the field of genetically modified enzyme and microorganism comprising such a modified enzyme for the production of raspberry ketone or zingerone. The modified enzyme is a recombinant benzalacetone synthase (BAS) issued or derived from a wild-type BAS. The modified BAS has the capability to produce 4-hydroxybenzalacetone from 4-coumaroyl-CoA in a more effective way as compared to wild-type BAS.

## Description

### Technical field

The present invention is in the field of genetically modified enzyme and microorganism comprising such a modified enzyme to produce raspberry ketone or zingerone. The modified enzyme is a recombinant benzalacetone synthase (BAS) issued or derived from a wild-type BAS. The modified BAS has the capability to produce 4-hydroxybenzalacetone from 4-coumaroyl-CoA, or 4-(4-hydroxy-3-methoxyphenyl)but-3-en-2-one from ferulic acid, in a more effective way as compared to wild-type BAS.

### Prior art

The bioproduction of "natural" flavors and fragrances has been an important area of research for the industry for many years in order to meet the needs of consumers who are increasingly concerned about being environmentally responsible. Synthetic biology, in particular through the use of microorganisms, allows this natural production, but the yields are not always sufficient for large-scale production.

The flavor of raspberry (*Rubus idaeus*) is linked to more than 200 compounds, but raspberry ketone (also known under the name raspinone or frambinone, these terms being interchangeably used herein), a naturally occurring phenolic compound, is the most impactful compound, defining its characteristic flavor (Klesk et al., 2004, J. Agric. Food Chem. 52, 5155- 61; Larsen et al., 1991, Acta Agric. Scand. 41, 447-54).

Since it is present only in small amounts in raspberries (1-4 mg per kg of fruit), natural raspberry ketone is of great value (Larsen et al., 1991). However, because its natural availability is limited, its biotechnological production is highly desirable. However, raspberry flavor is a major ingredient for flavor and flagrance industry , and is thus very much in demand.

Raspberry ketone is a polyketide. Polyketides represent a large family of metabolites produced by plants. Some of them are widely used in medicine, agriculture and cosmetic industry. Flavonoids, stilbenes, benzalacetones, are polyketides.

It is possible to produce synthetic raspberry ketone, but biosynthesis is nowadays preferable and especially gives a natural molecule unlike the petrochemical production route.

Other phenylpropanoid flavors, like zingerone which is a flavor component of ginger, are also of interest for production through biosynthesis. Zingerone is for example used as a flavor additive in spice oils and in perfumery to introduce spicy aromas.

The raspberry ketone and zingerone production pathways follow the phenylpropanoid production pathway in plants. Phenylpropanoids are key molecules for flavor production in most plants. The phenylpropanoid biosynthetic pathway can be reconstituted within a microorganism by inserting heterologous plant genes encoding some key enzymes of the pathway such as Tyrosine Ammonia Lyase ("TAL", catalyzing the transformation of Tyrosin into coumaric acid).

For example, raspberry ketone is produced from p-coumaroyl-CoA by a 2-step reaction catalyzed by a type III polyketide synthase with benzalacetone synthase (BAS) activity. This BAS enzyme catalyzes the condensation of a p-coumaroyl-CoA molecule with a malonyl-CoA molecule into a 4-hydroxybenzalacetone (Abe et al., 2001). The 4-hydroxybenzalacetone molecule (or "4-HBA") is then reduced to raspberry ketone by a benzalacetone reductase enzyme (or "BAR"). This pathway was recently implemented in the yeast *Saccharomyces cerevisiae* and resulted in the *de novo* production of raspberry ketone (Lee et al., 2016).

To sum up, Benzalacetone Synthases ("BAS" or "BAS enzyme") catalyze the transformation of coumaroyl-coA into 4-hydroxybenzalacetone, and 4-hydroxybenzalacetone is then metabolized to raspberry ketone by a benzalacetone reductase enzyme such as one issued from the raspberry *Rubus idaeus* (RZS/RKS).

A BAS enzyme exists in raspberry but BAS enzyme from *Rheum palmatum* (rhubarb) is the most commonly used in synthetic biology.

So far, the BAS enzymes from *Rheum palmatum, Wachendorfia thyrsiflora, Rubus ideaus* and *Polygonum cuspidatum* have been cloned and tested (Shimokawa et al., 2012). However, in all microorganisms where the raspberry ketone biosynthetic pathway has been implemented, the BAS enzyme from rhubarb (*Rheum palmatum*) is the one preferred (Wang et al., 2019; Milke et al., 2020; Chang et al., 2021).

Among the different studies on the activity of BAS, different analyses have been performed on the catalytic constant during the enzymatic reaction catalyzed by the BAS (Kₘ affinity and k_{cat} catalytic constant). For 4-coumaroyl-CoA, k_{cat}= 1.79 min-1 and affinity K_{M} = 10.0 µM and for malonyl-CoA k_{cat} = 1.78 min-1 and K_{M} = 23.3 µM. Thus, this enzyme is rather slow, which poses a problem of metabolic flux when inserting it into a biosynthetic pathway for biotechnological purposes (Abe et al., 2001; 2007; Morita et al., 2010).

The catalytic constants of raspberry or rhubarb BAS enzymes are very low compared to other enzymes used in the bioproduction pathway of raspberry ketone. The carbon flow is therefore slowed down by this enzyme, which does not allow an efficient conversion of the precursors (including coumaroyl-CoA) into benzalacetone derivatives (including 4-hydroxybenzalacetone compound and/or a raspberry ketone compound).

Thus, the BAS from *Rheum palmatum* is not very efficient, and not compatible with an efficient production of raspberry ketone or zingerone in fermentation. The development of more efficient BAS enzymes is crucial for the production of flavor compounds such as raspberry ketone or zingerone or others in microorganisms. Having more efficient BAS will lead to the provision of more efficient microorganism for producing flavor compounds such as raspberry ketone, zingerone or others. Having more efficient BAS will lead to the provision of enhanced processes for producing phenylpropanoid compounds, including 4-hydroxybenzalacetone compound and/or a raspberry ketone compound or 4-(4-Hydroxy-3-methoxyphenyl)but-3-en-2-one and/or a zingerone compound. Such an improved process would be more efficient and economically profitable with increased production yields of the final product raspberry ketone or zingerone.

Thus, there is a need to develop new enzymes and new microorganisms for the production of phenylpropanoid compounds, including 4-hydroxybenzalacetone and/or a raspberry ketone or 4-(4-Hydroxy-3-methoxyphenyl)but-3-en-2-one and/or a zingerone.

The object of the present invention is thus to provide a modified BAS enzyme, in particular modified BAS enzyme issued from *Rheum palmatum,* that has an enhanced catalytic activity as compared to wild-type BAS, in particular wild-type BAS enzyme from *Rheum palmatum.* One of the aims of the invention is to provide BAS enzyme and genetically modified microorganism comprising means for expressing such a modified BAS enzyme allowing a more efficient production of 4-hydroxybenzalacetone compound and/or a raspberry ketone compound or 4-(4-Hydroxy-3-methoxyphenyl)but-3-en-2-one and/or a zingerone compound, in particular from a 4-coumaroyl-CoA compound or from ferulic acid. In particular, there is a need for a BAS enzyme, that enhances the conversion of coumaroyl-CoA into benzalacetone 4-hydroxybenzalacetone and/or that enhances the conversion of ferulic acid into 4-(4-Hydroxy-3-methoxyphenyl)but-3-en-2-one.

### Summary of the invention

In a first aspect of the present invention, it is provided a modified BAS enzyme (also referenced as a "modified BAS" or "recombinant BAS") derived or issued from a wild-type BAS, in particular a wild-type BAS issued from *Rheum palmatum, Wachendorfia thyrsiflora, Rubus ideaus* or *Polygonum cuspidatum,* the modified BAS having a T281A substitution as compared to the wild-type BAS from which it is issued.

In another aspect of the present invention, it is provided a recombinant microorganism, in particular a recombinant *Pseudomonas putida,* capable to express or expressing a modified BAS enzyme according to the invention.

In another aspect, it is provided a genetically modified microorganism, in particular a recombinant microorganism, more particularly a recombinant *Pseudomonas putida,* capable to express or expressing a modified BAS enzyme according to the invention for the production of phenylpropanoid compounds, in particular 4-hydroxybenzalacetone compound and/or a raspberry ketone compound or 4-(4-Hydroxy-3-methoxyphenyl)but-3-en-2-one and/or a zingerone compound, more particularly 4-hydroxybenzalacetone compound and/or a raspberry ketone.

In another aspect, it is provided a modified BAS enzyme or a genetically modified microorganism, in particular a recombinant microorganism, more particularly a recombinant *Pseudomonas putida* capable of expressing or expressing a BAS enzyme according to the invention, for use in the production of phenylpropanoid compounds, in particular 4-hydroxybenzalacetone compound and/or a raspberry ketone compound or 4-(4-Hydroxy-3-methoxyphenyl)but-3-en-2-one and/or a zingerone compound, more particularly 4-hydroxybenzalacetone compound and/or a raspberry ketone compound.

In another aspect, it is provided a process for the synthesis of phenylpropanoid compounds, in particular 4-hydroxybenzalacetone compound and/or a raspberry ketone compound or 4-(4-Hydroxy-3-methoxyphenyl)but-3-en-2-one and/or a zingerone compound, by using a BAS enzyme according to the invention, and/or by using a genetically modified microorganism, in particular a recombinant microorganism, more particularly a recombinant *Pseudomonas putida,* capable to express or expressing a modified BAS enzyme according to the invention.

The invention is defined in the independent claims. Dependent claims define preferred embodiments. The features set forth in the following paragraphs may optionally be implemented. They can be implemented independently of each other or in combination with each other.

### Short description of the drawings

These and further aspects of the invention will be explained in greater detail by way of examples and with reference to the accompanying drawings in which:
**Fig. 1**

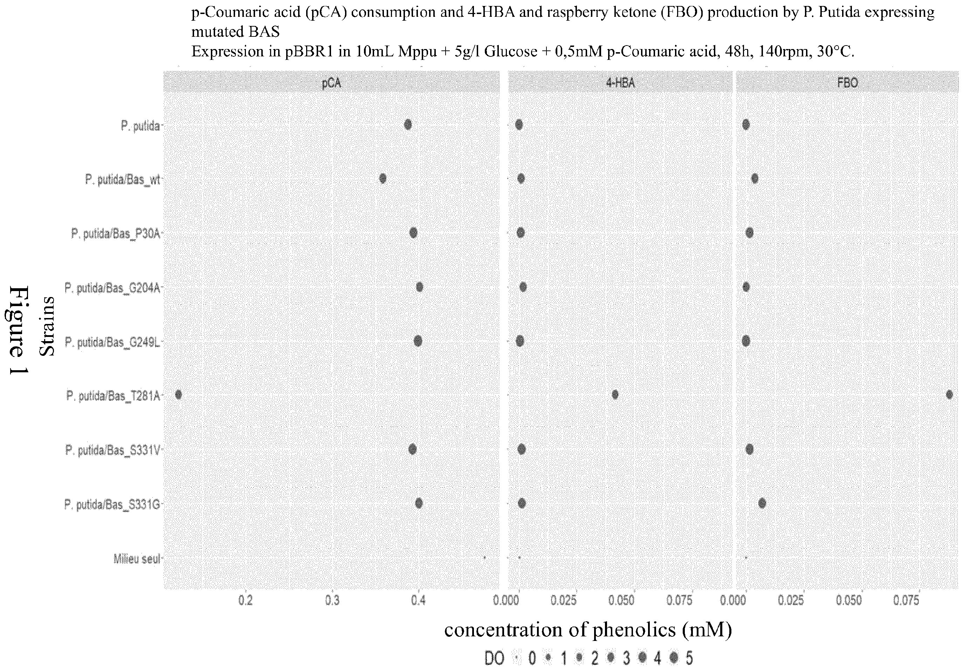

   Fig. 1 is a graph illustrating the concentration of p-Coumaric acid (pCA), 4-hydroxybenzalacetone (HBA) and raspberry ketone (FBO) in supernatants of cultured *P. putida.* The abscissa axis represents the concentration in mM of pCA (Fig. 1, left side), HBA (Fig. 1, middle) and FBO (right side). On the ordinate axis, different modified BAS enzymes are listed, wherein the modified BAS are defined by their mutation as compared to the wild-type *Rheum palmatum* BAS.
**Fig. 2**

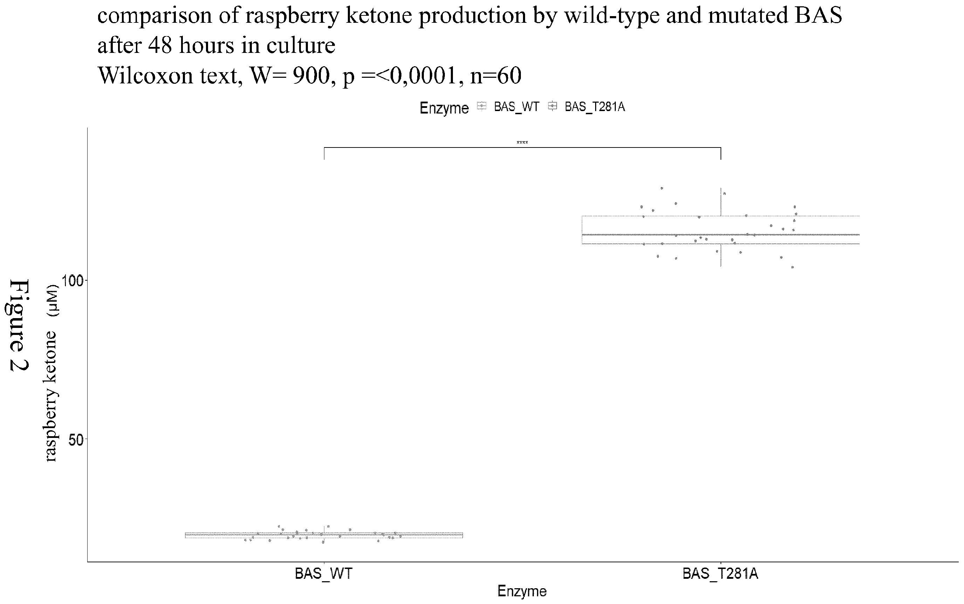

   Fig. 2 is a graph illustrating the production of raspberry ketone by recombinant *Pseudomonas putida* expressing wild-type *Rheum palmatum* BAS or recombinant *Pseudomonas putida* expressing a modified T281A *Rheum palmatum* BAS. Ordinate axis corresponds to the concentration of raspberry ketone expressed in µM in the supernatant of cultured *Pseudomonas putida.*
**Fig. 3**

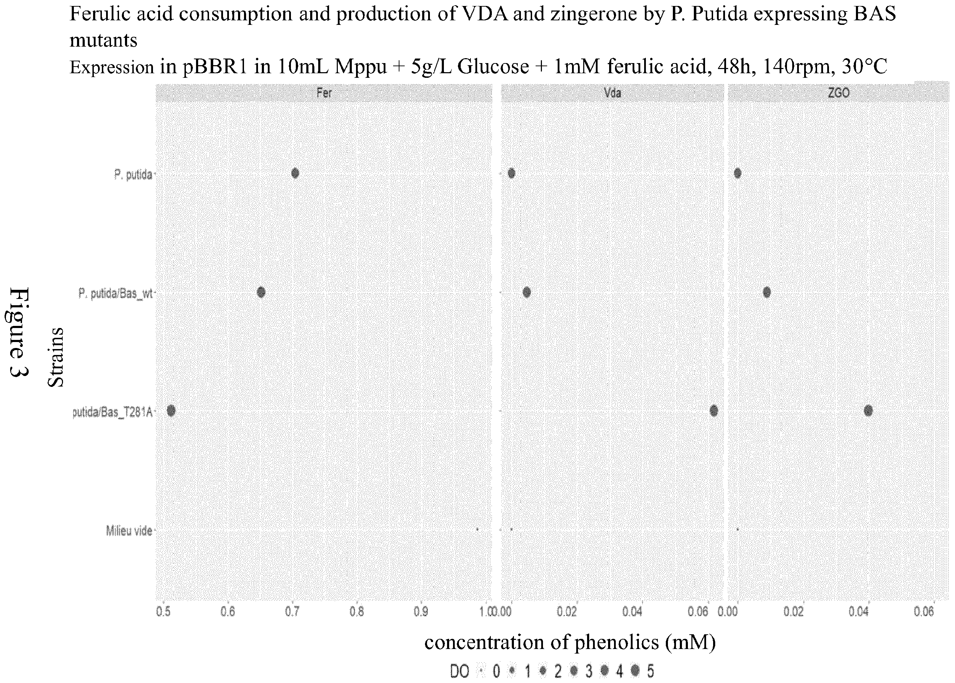

   Fig. 3 is a graph illustrating the concentration of Ferulic acid (Fer), 4-(4-Hydroxy-3-methoxyphenyl)but-3-en-2-one (Vda) and zingerone (ZGO) in supernatants of cultured *P. putida.* The abscissa axis represents the concentration in mM of Fer (Fig. 3, left side), Vda (Fig. 3, middle) and ZGO (Fig. 3, right side). On the ordinate axis, different modified BAS enzymes are listed, wherein the modified BAS are defined by their mutation as compared to the wild-type *Rheum palmatum* BAS

### Detailed description of embodiments of the invention

In a first aspect, it is provided a modified benzalacetone synthase enzyme (BAS) issued or derived from a wild-type BAS, the recombinant BAS having a T281A substitution as compared to the wild-type BAS from which it is issued or derived. Such a modified BAS has the capability to convert p-coumaroyl-CoA into 4-hydroxybenzalcetone and/or ferulic acid into 4-(4-Hydroxy-3-methoxyphenyl)but-3-en-2-one. Such a modified BAS converts p-coumaroyl-CoA into 4-hydroxybenzalcetone and/or ferulic acid into 4-(4-Hydroxy-3-methoxyphenyl)but-3-en-2-one in a more efficient manner than the wild-type BAS from which it is issued or derived.

In a particular embodiment of the invention, the modified BAS has an improved catalytic activity on the transformation of 4-coumaroyl-CoA into 4-hydroxybenzalacetone and/or ferulic acid into 4-(4-Hydroxy-3-methoxyphenyl)but-3-en-2-one as compared to the wild-type BAS from which it is issued or derived.

The modified BAS may be obtained by genetic engineering of the wild-type BAS from which it is issued or derived, or from a gene or nucleic acid molecule encoding such a wild-type BAS. One or more non-natural mutations can be introduced into the modified BAS, for example by insertion, substitution or deletion of nucleotides encoding the wild-type BAS, said mutations being obtained by transformation techniques or by gene editing techniques known to the skilled artisan.

Genetic modification techniques by transformation, mutagenesis or gene editing are well known to the skilled person and are described for example in "Strategies used for genetically modifying bacterial genome: site-directed mutagenesis, gene inactivation", Journal of Zhejiang Univ-Sci B (Biomed & Biotechnol) 2016 17(2):83-99, and in Martinez-Garcia and de Lorenzo, "Pseudomonas putida in the quest of programmable chemistry", Current Opinion in Biotechnology, 59 :111-121, 2019.

In a particular aspect of the invention, the wild-type BAS from which the modified BAS is issued or derived is from *Rheum palmatum, Wachendorfia thyrsiflora, Rubus ideaus* or *Polygonum cuspidatum.* In a more particular embodiment, the wild-type BAS from which the modified BAS is issued or derived is from *Rheum palmatum.* More particularly, the wild-type BAS from which the modified BAS is issued or derived has the amino acid sequence set forth in SEQ ID No. 1. In an embodiment, the wild-type BAS is encoded by the nucleotide sequence set forth in SEQ ID No. 5. In an embodiment, the BAS (either wild-type or the modified one) is encoded within a vector, in particular a plasmid, having the nucleotide sequence set forth in SEQ ID No. 6.

By issued or derived, it should be understood that the BAS of the invention is modified as compared to the wild-type BAS by mutation, including by substitution (including conservative amino acid residue(s)) or by addition and/or deletion of amino acid residues or by secondary modification after translation or by deletion of portions of the wild-type proteins(s) resulting in fragments having a shortened size with respect to the wild-type protein of reference. Fragments of the wild-type BAS are encompassed within the present invention to the extent that they possess the capability to transform 4-coumaroyl-CoA into 4-hydroxybenzalacetone and/or ferulic acid into 4-(4-Hydroxy-3-methoxyphenyl)but-3-en-2-one, and the T281A substitution as compared to the wild-type BAS.

In particular, a modified BAS may be obtained by modifying a nucleic acid that encodes a wild-type BAS by the method detailed in example 1.

In a particular aspect of the invention, the modified BAS of the invention has an improved catalytic activity on the condensation of a p-coumaroyl-CoA molecule with a malonyl-CoA molecule into a 4-hydroxybenzalacetone as compared to the catalytic activity of the wild-type BAS from which it is issued or derived. In a more particular aspect, the modified BAS of the invention improves at least 2, 3, 4, 5, 6 or 7 times more the condensation of a p-coumaroyl-CoA molecule with a malonyl-CoA molecule into a 4-hydroxybenzalacetone as compared to the catalytic activity of the wild-type BAS from which it is issued or derived.

The catalytic activity of the BAS may be determined by measuring the catalytic constant k_{cat} of the BAS in a condensation reaction of a p-coumaroyl-CoA molecule with a malonyl-CoA molecule into a 4-hydroxybenzalacetone. It can be calculated from the maximum reaction rate and catalyst site concentration by methods known by the skilled artisan.

In a particular embodiment, the modified BAS of the invention has at least the same catalytic activity, in particular at least the same catalytic constant k_{cat}, on the condensation reaction of a p-coumaroyl-CoA molecule with a malonyl-CoA molecule into a 4-hydroxybenzalacetone, as compared to a BAS having the amino acid sequence set forth in SEQ ID No. 2.

In an embodiment of the invention, the modified BAS of the invention has an amino acid sequence selected from the sequences set forth in the group consisting of SEQ ID No. 2; SEQ ID No. 7; SEQ ID No. 8; SEQ ID No. 9; SEQ ID No. 10; SEQ ID No. 11; SEQ ID No. 12; SEQ ID No. 13; SEQ ID No. 14; SEQ ID No. 15; SEQ ID No. 16; SEQ ID No. 17; SEQ ID No. 18; SEQ ID No. 19; SEQ ID No. 20; SEQ ID No. 21; SEQ ID No. 22; SEQ ID No. 23; SEQ ID No. 24; SEQ ID No. 25, SEQ ID No. 26; SEQ ID No. 27; SEQ ID No. 28 and SEQ ID No. 29.

In a particular embodiment, the modified BAS of the invention is issued or derived from a wild-type BAS, the modified BAS having a T281A substitution as compared to the wild-type BAS from which it is issued or derived, and is a functional equivalent of the BAS having the amino acid sequence set forth in SEQ ID No. 2.

In a particular embodiment, the modified BAS of the invention is issued or derived from a wild-type BAS, the modified BAS having a T281L substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. The Ptrc promoter is a hybrid of the lac and trp promoters. In particular, the modified BAS has the amino acid sequence set forth in SEQ ID No. 7.

In a particular embodiment, the modified BAS of the invention is issued or derived from a wild-type BAS, the modified BAS having a T281I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 8.

In a particular embodiment, the modified BAS of the invention is issued or derived from a wild-type BAS, the modified BAS having a T281I substitution and a K105N substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 9.

In a particular embodiment, the modified BAS of the invention is issued or derived from a wild-type BAS, the modified BAS having a T281I substitution and a G111D substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 10.

In a particular embodiment, the modified BAS of the invention is issued or derived from a wild-type BAS, the modified BAS having a T281C substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 11.

In a particular embodiment, the modified BAS of the invention is issued or derived from a wild-type BAS, the modified BAS having a T281K substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 12.

In a particular embodiment, the modified BAS of the invention is issued or derived from a wild-type BAS, the modified BAS having a substitution of its Threonine localized at position 281 for any other amino acid residue (e.g. T281X substitution, wherein X can be any amino acid residue that is not T) as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence.

In a particular embodiment, the modified BAS of the invention is issued or derived from a wild-type BAS, the modified BAS having a T281K substitution and a V264I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 13.

In a particular embodiment, the modified BAS of the invention is issued or derived from a wild-type BAS, the modified BAS having a L141I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the P14g_RBS005 promoter sequence, or the Ptrc promoter sequence, or the P14g_RBS005 and the Ptdr promoter sequences. In particular, the modified BAS has the amino acid sequence set forth in SEQ ID No. 14.

In a particular embodiment, the modified BAS of the invention is issued or derived from a wild-type BAS, the modified BAS having a L141I substitution and a T281A substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 15.

In a particular embodiment, the modified BAS of the invention is issued or derived from a wild-type BAS, the modified BAS having a R51K substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 16.

In a particular embodiment, the modified BAS of the invention is issued or derived from a wild-type BAS, the modified BAS having a N85D substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 17.

In a particular embodiment, the modified BAS of the invention is issued or derived from a wild-type BAS, the modified BAS having a M186N substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 18.

In a particular embodiment, the modified BAS of the invention is issued or derived from a wild-type BAS, the modified BAS having a M186V substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 19.

In a particular embodiment, the modified BAS of the invention is issued or derived from a wild-type BAS, the modified BAS having a T189C substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 20.

In a particular embodiment, the modified BAS of the invention is issued or derived from a wild-type BAS, the modified BAS having a T189I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 21.

In a particular embodiment, the modified BAS of the invention is issued or derived from a wild-type BAS, the modified BAS having a T189V substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 22.

In a particular embodiment, the modified BAS of the invention is issued or derived from a wild-type BAS, the modified BAS having a G209A substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 23.

In a particular embodiment, the modified BAS of the invention is issued or derived from a wild-type BAS, the modified BAS having a G209S substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 24.

In a particular embodiment, the modified BAS of the invention is issued or derived from a wild-type BAS, the modified BAS having a S269A substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 25.

In a particular embodiment, the modified BAS of the invention is issued or derived from a wild-type BAS, the modified BAS having a A295V substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 26.

In a particular embodiment, the modified BAS of the invention is issued or derived from a wild-type BAS, the modified BAS having a V264I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 27.

In a particular embodiment, the modified BAS of the invention is issued or derived from a wild-type BAS, the modified BAS having a V264I substitution and a L141I substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 28.

In a particular embodiment, the modified BAS of the invention is issued or derived from a wild-type BAS, the modified BAS having a L141I substitution, a V264I substitution and a T281A substitution as compared to the wild-type BAS from which it is issued or derived, and is a functional equivalent of the BAS having the amino acid sequence set forth in SEQ ID No. 2. In a preferred embodiment, the expression of the modified BAS is under the control of the Ptrc promoter sequence. In particular, the modified BAS has the amino acid sequence set forth in SEQ ID No. 29.

A functional variant of the BAS having the amino sequence set forth in SEQ ID No. 2 is a BAS having a polypeptide sequence which is issued or derived from the polypeptide sequence of one wild-type BAS, in particular a wild-type BAS from *Rheum palmatum, Wachendorfia thyrsiflora, Rubus ideaus* or *Polygonum cuspidatum,* or the BAS having the amino acid sequence set forth in SEQ ID No. 2, which may comprise further modification(s), i.e. substitution(s), insertion(s) and/or deletion(s) of one or more amino acids but which retains the activity of the BAS having the amino acid sequence set forth in SEQ ID No. 2, and in particular the capability to convert p-coumaroyl-CoA into 4-hydroxybenzalcetone as described above with at least the same catalytic activity. Preferably but not necessarily, the functional equivalent still has the T281A substitution as compared to the wild-type protein from which it is issued. Fragments may have a size representing at least 50% of the amino-acid sequence size of the wild-type BAS in particular at least 60%, or at least 70%, or at least 80%, or at least 90%, or at least 95%.

The catalytic activity of a functional variant of the BAS having the amino acid sequence set forth in SEQ ID No. 2 can be assessed by any method known to the skilled person, in particular as illustrated in the example of the invention, by expressing in a strain of *Pseudomonas putida* a recombinant gene encoding the functional variant of the BAS having the amino acid sequence set forth in SEQ ID No. 2, preferably cloned into a plasmid downstream of a promoter allowing its expression in the strain, and culturing the strain in the presence of p-coumaroyl-CoA and assaying by HPLC the total concentration of 4-hydroxybenzalcetone and/or raspberry ketone, preferably 4-hydroxybenzalcetone, produced by the strain after 24h. The functional variant maintains at least the same BAS activity as compared to the BAS activity measured when the BAS having the amino acid sequence set forth in SEQ ID No. 2 is expressed in the strain of *Pseudomonas putida* in the same experimental condition. The BAS activity is determined according to the amount or concentration of 4-hydroxybenzalcetone and/or raspberry ketone, preferably 4-hydroxybenzalcetone, produced by the strain during a determined period.

BAS activity may be determined by measuring the quantity of 4-hydroxybenzalcetone by HPLC in the following test:
In a 200µl medium comprising:
   - 0.5mM of Coumaric acid;1mM of coenzyme A,
   - 5mM of ATP,
   - 25mM of MgCl₂;
   - 1mM of malonyl-CoA,
   - 500mM of Tris-HCl buffer at pH 7.5;
   - 50µg of 4-coumarate--CoA ligase 1,
100µg/ml of the BAS to be tested is added.

The medium with the BAS is incubated during 24 hours at 30°C.

After this 24h period, the enzymatic reaction is stopped by adding 50mM of HCl.

The production of 4-hydroxybenzalcetone is measured by HPLC.

A BAS is considered to be a functional variant of the BAS of SEQ ID No. 2 when the production of 4-hydroxybenzalcetone in presence of the tested BAS is at least equal to the production of 4-hydroxybenzalcetone in presence of the BAS of SEQ ID No. 2.

Preferably, a functional variant of the BAS having the amino acid sequence set forth in SEQ ID No. 2 corresponds to a polypeptide sequence having at least 80%, 85%, 90%, 95% and most particularly, at least 98% identity with one of the sequences selected from SEQ ID No. 1 or SEQ ID No. 2. In a preferred embodiment, the T281A substitution is present as compared to the wild-type protein from which the functional equivalent is issued. A percentage identity refers to the percentage of identical residues in a nucleotide or amino acid sequence on a given fragment after alignment and comparison with a reference sequence. For the comparison, an alignment algorithm is used and the sequences to be compared are entered with the corresponding parameters of the algorithm. The default parameters of the algorithm can be use

In a particular embodiment, for a nucleic acid or polypeptide sequence comparison and determination of a percent identity, the blastn or blastp algorithm as described in https://blast.ncbi.nlm.nih.gov/Blast.cgiavec is used, in particular with default parameters. In particular, the functional variant refers to a polypeptide that has an amino acid sequence that differs from one of the sequences selected from SEQ ID NO: 1 or SEQ ID No. 2 by less than 50, 40, 30, 20, 10, 5, 4, 3, 2 or 1 substitutions, insertions or deletions

In another particular embodiment, the functional variant refers to a polypeptide that has an amino acid sequence that differs from one of the sequences selected from SEQ ID NO: 1 or SEQ ID No. 2 by fewer than 50, 40, 30, 20, 10, 5, 4, 3, 2 or 1 substitutions, the substitutions preferably being conservative substitutions. The term "conservative substitution" as used herein refers to the replacement of one amino acid residue with another, without altering the conformation or enzymatic activity of the polypeptide so modified, including, but not limited to, the replacement of one amino acid with another having similar properties (such as, for example, polarity, hydrogen bonding potential, acidity, basicity, shape, hydrophobicity, aromaticity and the like).

Examples of conservative substitutions are found in the groups of basic amino acids (arginine, lysine and histidine), acidic amino acids (glutamic acid and aspartic acid), polar amino acids (glutamine and asparagine), hydrophobic amino acids (methionine, leucine, isoleucine and valine), aromatic amino acids (phenylalanine, tryptophan and tyrosine) and small amino acids (glycine, alanine, serine and threonine).

In a particular embodiment, the modified BAS enzyme of the invention has the amino acid sequence set forth in SEQ ID No. 2.

In a particular embodiment, the modified BAS enzyme is a recombinant protein.

In a particular embodiment, the wild type BAS enzyme is issued from *Rheum palmatum.*

In a particular embodiment, the wild type BAS enzyme is issued from *Rheum palmatum* and has the amino acid sequence set forth in SEQ ID No. 1.

In another embodiment, it is provided a nucleic acid molecule encoding a BAS enzyme according to the invention. In a preferred embodiment, the nucleic acid encodes the BAS according to any embodiment disclosed herein. In a particular embodiment, the nucleic acid molecule that encodes a modified BAS enzyme according to the invention has the sequence set forth in SEQ ID No. 3. In a particular embodiment, the nucleic acid molecule that encodes a modified BAS enzyme according to the invention is a plasmid, in particular is a plasmid comprising the sequence set forth in SEQ ID No. 3 or comprising or consisting of the sequence set forth in SEQ ID No. 4.

The invention also relates to a nucleic acid molecule encoding the BAS as disclosed herein, or comprising the nucleic acid molecule(s) as disclosed herein. Encompassed within the invention is a vector encoding the BAS as disclosed herein. As used herein, a vector is a nucleic acid molecule used as a vehicle to transfer a genetic material into a cell, and in a preferred embodiment allows the expression of a gene encoding the BAS inserted within the vector. The term vector encompasses plasmids, viruses, cosmids and artificial chromosomes. The vector itself is generally a nucleotide sequence, commonly a DNA sequence, that comprises an insert (a transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene (*i*.*e*. the gene encoding the modified BAS of the invention) in a target cell (i.e. a genetically engineered *Pseudomonas putida*)*,* and generally have control sequences.

In another embodiment of the invention, the applicant has developed a strain of Pseudomonas putida capable of expressing a benzalacetone synthase to more efficiently produce a 4-hydroxybenzalketone from a p-coumaroyl-coA according to the reaction as described above.

According to another particular embodiment, the genetically modified *Pseudomonas putida* strain comprises an additional recombinant gene encoding a polypeptide with benzalacetone synthase (BAS) activity. In particular, the modified strain may comprise a recombinant gene encoding a BAS (EC 2.3.1.212) whose sequence is defined by the amino acid sequence SEQ ID NO: 3 or by a sequence having at least 80%, 85%, 90%, 95% and most particularly, at least 98% identity with the sequence SEQ ID NO: 3 and encoding a BAS according to the invention.

According to a particular embodiment, the genetically modified *Pseudomonas putida* strain comprises an additional recombinant gene encoding a polypeptide with benzalacetone synthase (BAS) activity comprising or having or consisting of the nucleotide sequence set forth in SEQ ID No. 3.

According to a particular embodiment, the genetically modified *Pseudomonas putida* strain comprises a vector, in particular a plasmid, more particularly a plasmid comprising or having or consisting of the nucleotide sequence set forth in SEQ ID No. 4, the vector thus comprising an additional recombinant gene encoding a polypeptide with benzalacetone synthase (BAS) activity comprising or having or consisting of the nucleotide sequence set forth in SEQ ID No. 3.

According to this particular embodiment, the genetically modified strain is capable of converting p-coumaroyl-coA to 4-hydroxybenzalketone via the BAS enzyme.

Thus, an object of the invention relates to a genetically modified strain of Pseudomonas putida characterized in that it is capable of expressing or expresses a recombinant gene encoding a benzalacetone synthase capable of producing 4-hydroxybenzalacetone from p-coumaroyl-coA.

In a preferred embodiment, the *Pseudomonas putida* strain according to the present application is capable of producing 4-hydroxybenzalacetone from p-coumaroyl-CoA.

In another preferred embodiment, the *Pseudomonas putida* strain according to the present application is capable of producing raspberry ketone from 4-hydroxybenzalacetone.

In another preferred embodiment, the *Pseudomonas putida* strain according to the present application is capable of producing zingerone from 4-(4-Hydroxy-3-methoxyphenyl)but-3-en-2-one.

p-coumaroyl-CoA or 4-coumaroyl-CoA is a compound of formula (I):

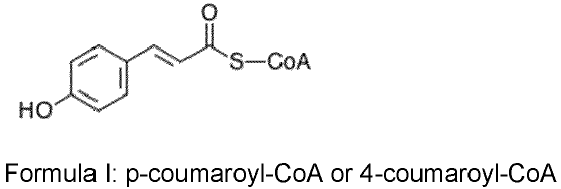

4-hydroxybenzalacetone is a compound of formula (II):

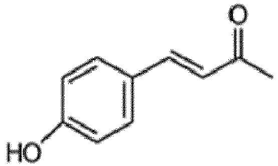

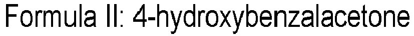

Raspberry ketone is a natural phenolic compound of IUPAC name 4-(4-Hydroxyphenyl)butan-2-one and of formula (III):

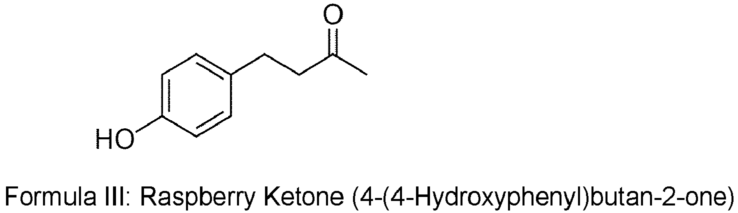

Ferulic acid or (2E)-3-(4-hydroxy-3-methoxyphenyl)prop-2-enoic acid (IUPAC name) is a compound of formula (IV):

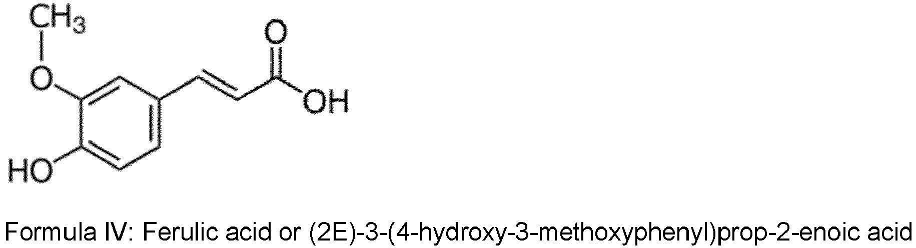

4-(4-hydroxy-3-methoxyphenyl)but-3-en-2-one or (E)-4-(4-hydroxy-3-methoxyphenyl)but-3-en-2-one (IUPAC name) is a precursor of zingerone. It is also referenced as Vanillylidene acetone (Vda) in literature and in the working example of the invention. 4-(4-hydroxy-3-methoxyphenyl)but-3-en-2-one is a compound of formula (V):

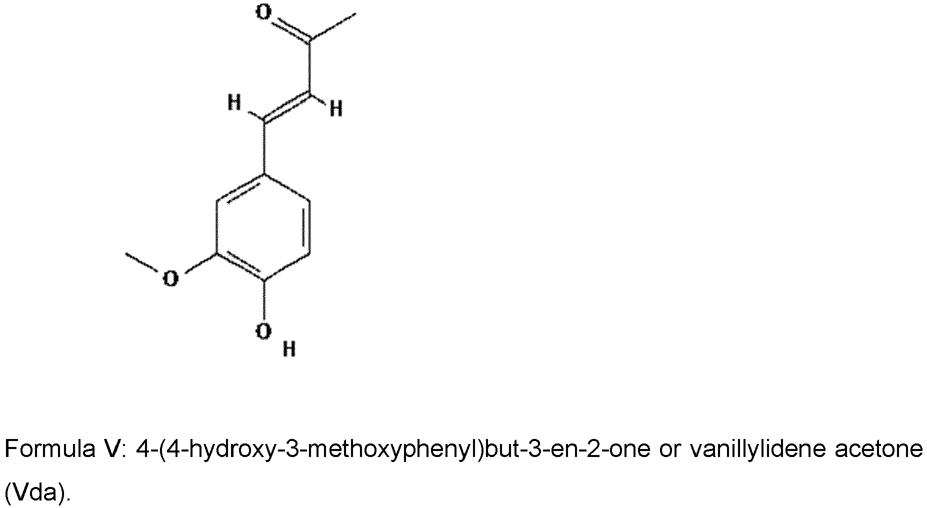

Zingerone, also called vanillylacetone, is a flavor component of ginger. Zingerone is also known under IUPAC name 4-(4-Hydroxy-3-methoxyphenyl)butan-2-one. Zingerone is a compound of formula (VI):

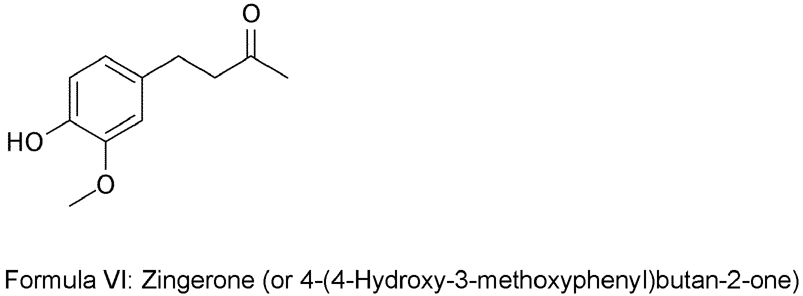

In another preferred embodiment, said genetically modified Pseudomonas putida strain is characterized in that it expresses a recombinant gene encoding a benzalacetone synthase according to the invention and at least one gene encoding a benzalacetone reductase, in particular selected from the group consisting of the NADPH-dependent 2-alkenal reductase of arabidopsis thaliana (Uniprot Q39172, updated June 2, 202, the ene-reductase from Zingiber officinale (Uniprot A0A096LNF0, updated on April 7, 2021), the NADPH-dependent curcumin reductase, also called CurA from Pseudomonas Putida (Uniprot Q88K17, updated on December 2, 2020) NADP-dependent alkenal double bond reductase, also known as DBR from Olimarabidopsis pumila (Uniprot A0A1C9CX65, updated August 12, 2020), NADP(+)-dependent 2-alkenal reductase, also known as DBR from Nicotiana tabacum (Uniprot Q9SLN8 ; EC 1. 3.1.102), or the NADP(+)-dependent 2-alkenal reductase, also known as Red from Capsicum annuum (Uniprot A0A1U8GFY1, updated 10 February 2021).

In a particular embodiment, the invention relates to a genetically modified strain of *Pseudomonas putida* characterized in that it expresses a recombinant gene encoding a functional equivalent of a previously described benzalacetone synthase.

Wild type strains of *Pseudomonas. putida* KT2440 are available for example in the NBRC Strain Bank (National Institute of Technology and Evaluation Biological Resource center https://www.nite.go.jp/en/nbrc/, NBRC100650).

Furthermore, strains of *Pseudomonas putida* or *Pseudomonas taiwanensis* optimized for tyrosine production are known to the skilled person who will be able to use them as starting strains to obtain the genetically modified strains according to the present invention (Calero et al., ACS Synth Biol. 2016 Jul 15;5(7):741-53; Wierckx et al, Appl Environ Microbiol. 2005 Dec;71(12):8221-7, Appl Environ Microbiol. 71(12):8221-7; Wynands et al, 2018; Otto et al 2019, Front Bioeng Biotechnol Nov 20;7:312).

As used in this description, the terms "genetically modified (*Pseudomonas putida*) strain," "modified (*Pseudomonas putida*) strain," or "genetically modified strain," are considered synonymous with each other.

In particular, "genetically modified strain" is understood to mean a strain that comprises either (i) at least one recombinant nucleic acid, or transgene, stably integrated into its genome, and/or present on a vector, e.g., a plasmid vector, or (ii) one or more unnatural mutations by nucleotide insertion, substitution, or deletion, said mutations being obtained by transformation techniques or by gene editing techniques known to the skilled person. In a particular embodiment, a genetically modified strain is a strain having stably integrated into its genome at least one exogenous nucleic acid, *i*.*e*. not naturally present in P. putida, for example a nucleic acid from another species, for example from *Rheum palmatum, Wachendorfia thyrsiflora, Rubus ideaus or Polygonum cuspidatum.*

For the purposes of the present invention, "recombinant gene encoding a benzalacetone synthase " means an exogenous nucleic acid comprising at least a portion encoding a benzalacetone synthase according to the invention as described above. In addition to the region encoding the benzalacetone synthase, the recombinant gene may be under the control of a promoter enabling its expression in the strain, preferably a promoter enabling its expression in the *P. putida* strain.

In a particular embodiment, the nucleic acid encoding a BAS according to the invention comprises a nucleotide sequence according to SEQ ID No. 3 or a sequence having at least 80%, 85%, 90%, 95% and most preferably at least 98% identity with a sequence of SEQ ID No. 3.

In one embodiment, which may be combined with the preceding ones, the recombinant gene encoding benzalacetone synthase as described above is placed under the control of a heterologous promoter, in particular a constitutive or inducible promoter, for example selected from among the ptrc, xyls/pm or araC/pBAD promoters, which allows the recombinant gene encoding benzalacetone synthase to be overexpressed in the genetically modified strain according to the invention.

The techniques of genetic modification by transformation, mutagenesis or gene editing are well known to the person skilled in the art and are described, for example, in "Molecular cloning: a laboratory manual", J. Sambrook, ed. Cold Spring Harbor, "Strategies used for genetically modifying bacterial genome: site-directed mutagenesis, gene inactivation," Journal of Zhejiang Univ-Sci B (Biomed & Biotechnol) 2016 17(2):83-99. and in Martinez-Garcia and de Lorenzo, "Pseudomonas putida in the quest of programmable chemistry," Current Opinion in Biotechnology, 59:111-121, 2019.

In one embodiment, a genetically engineered strain may comprise an expression-modifying nucleic acid, preferably overexpressing the expression of one or more genes naturally expressed in Pseudomonas putida.

Overexpression of a gene is understood as a higher expression of said gene in a genetically modified strain compared to the same strain in which the gene is expressed only under the control of the natural promoter. Overexpression can be achieved by inserting one or more copies of the gene directly into the genome of the strain, preferably under the control of a strong promoter, or also by cloning into plasmids, in particular multicopy plasmids, preferably also under the control of a strong promoter.

In another particular mode, a genetically modified strain may comprise a nucleic acid encoding one or more enzymes not naturally expressed in *Pseudomonas putida.*

Most advantageously, the Applicant has developed a Pseudomonas putida strain capable of expressing a benzalacetone synthase and efficiently producing 4-hydroxybenzalacetone.

Preferably, the Pseudomonas putida strain according to the present disclosure is capable of overproducing a raspberry ketone from 4-hydroxybenzalacetone.

Preferably, the Pseudomonas putida strain according to the present disclosure is capable of overproducing 4-hydroxybenzalacetone from 4-coumaroyl-CoA.

Preferably, the Pseudomonas putida strain according to the present disclosure is capable of overproducing raspberry ketone from 4-coumaroyl-CoA.

Preferably, the Pseudomonas putida strain according to the present disclosure is capable of overproducing a zingerone from 4-(4-Hydroxy-3-methoxyphenyl)but-3-en-2-one.

Preferably, the Pseudomonas putida strain according to the present disclosure is capable of overproducing 4-(4-Hydroxy-3-methoxyphenyl)but-3-en-2-one from ferulic acid.

Preferably, the Pseudomonas putida strain according to the present disclosure is capable of overproducing a zingerone from ferulic acid.

In a particular embodiment of the invention, there is provided an overproducing strain of 4-hydroxybenzalacetone and/or raspberry ketone or 4-(4-Hydroxy-3-methoxyphenyl)but-3-en-2-one and/or zingerone. By "overproducing strain" in the sense of the present disclosure is meant a modified Pseudomonas putida strain capable of producing a 4-hydroxybenzalacetone and/or a raspberry ketone or 4-(4-Hydroxy-3-methoxyphenyl)but-3-en-2-one and/or zingerone in a higher amount compared to a wild type Pseudomonas putida strain.

Preferably, the overproducing strain according to the present disclosure is capable of producing 2, 3, 4, 5 or 6 times more 4-hydroxybenzalacetone and/or raspberry ketone or 4-(4-Hydroxy-3-methoxyphenyl)but-3-en-2-one and/or zingerone compared to a wild-type *Pseudomonas putida* strain or a Pseudomonas putida strain expressing a wild type BAS as shown in the examples.

Preferably, the overproducing strain is capable of converting all of the 4-coumaroyl-CoA in situ or added to the culture medium, to 4-hydroxybenzalacetone, and preferably converting all of the 4-hydroxybenzalacetone to raspberry ketone or all of 4-(4-Hydroxy-3-methoxyphenyl)but-3-en-2-one to zingerone .

According to another particular embodiment, the strain according to the present application is capable of producing raspberry ketone from 4-hydroxybenzalacetone or zingerone from 4-(4-Hydroxy-3-methoxyphenyl)but-3-en-2-one (*in situ* synthesis). The strain may thus further comprise at least one additional recombinant gene enabling the synthesis of raspberry ketone or zingerone, preferably a recombinant gene encoding a benzalacetone reductase.

By the term "additional recombinant gene" is meant in the sense of the present invention any recombinant gene present in the Pseudomonas putida strain in addition to the recombinant gene encoding a benzalacetone synthase according to the invention.

The additional recombinant gene may result from the insertion of a heterologous promoter, for example a strong promoter to overexpress an endogenous Pseudomonas putida gene, or a recombinant coding sequence encoding a protein not naturally expressed in Pseudomonas putida.

According to a particular embodiment, the genetically modified Pseudomonas putida strain comprises an additional recombinant gene encoding a tyrosine ammonia lyase (TAL) active polypeptide. A recombinant gene encoding TAL can be derived from the microorganism *Rhodotorula glutinis* and optimized according to reference Zhou et al, Appl Microbiol Biotechnol. 2016 Dec;100(24):10443-10452 (three point mutations in this TAL enzyme make it more efficient: S9N; A11T; E518V). This TAL enzyme is called TAL_rg_opt. In particular, the modified strain may comprise a recombinant gene encoding a tyrosine ammonia lyase (TAL) (EC 4.3.1.23).

According to this particular embodiment, the genetically modified strain according to the invention is capable of converting tyrosine to coumaric acid via the TAL enzyme.

According to another particular embodiment that can be combined with the previous one, the genetically modified strain of Pseudomonas putida comprises an additional recombinant gene encoding 4-coumarate-CoA ligase (4-CL). In particular, the modified strain may comprise a recombinant gene encoding a 4-CL (EC 6.2.1.12).

According to this particular embodiment, the genetically modified strain is capable of converting coumaric acid to p-coumaryl-coA via the 4-CL enzyme.

According to a preferred embodiment, the genetically modified Pseudomonas putida strain comprises one or several additional recombinant gene(s), namely one, two or the three following additional recombinant genes:
- a recombinant gene encoding a tyrosine ammonia lyase (TAL), and/or
- a recombinant gene encoding a 4-coumarate-CoA ligase (4-CL), and/or
- a recombinant gene coding for a benzalacetone reductase (BAR).

The TAL, 4-CL and BAR enzymes are all enzymes involved in the synthesis of phenylpropanoid compounds. According to this preferred embodiment, the modified strain is capable of producing a multitude of phenylpropanoid compounds, namely coumaric acid, p-coumaroyl-coA, 4-hydroxybenzalcetone, and raspberry ketone or 4-(4-Hydroxy-3-methoxyphenyl)but-3-en-2-one and/or zingerone.

Another object of the invention relates to the use of a BAS according to the invention for producing 4-hydroxybenzalcetone, or raspberry ketone, or 4-hydroxybenzalcetone and raspberry ketone.

Another object of the invention relates to the use of a BAS according to the invention for producing 4-(4-Hydroxy-3-methoxyphenyl)but-3-en-2-one, or zingerone, or 4-(4-Hydroxy-3-methoxyphenyl)but-3-en-2-one and zingerone.

According to a particular embodiment of the invention, it is provided a genetically modified strain of *Pseudomonas putida* as defined above, for use in the synthesis of 4-hydroxybenzalcetone, or raspberry ketone, or 4-hydroxybenzalcetone and raspberry ketone.

According to a particular embodiment of the invention, it is provided a genetically modified strain of *Pseudomonas putida* as defined above, for use in the synthesis of 4-(4-Hydroxy-3-methoxyphenyl)but-3-en-2-one, or zingerone, or 4-(4-Hydroxy-3-methoxyphenyl)but-3-en-2-one and zingerone.

Another object of the invention relates to a process for the synthesis of 4-hydroxybenzalcetone, or raspberry ketone, or 4-hydroxybenzalcetone and raspberry ketone.

Another object of the invention relates to a process for the synthesis of 4-(4-Hydroxy-3-methoxyphenyl)but-3-en-2-one, or zingerone, or 4-(4-Hydroxy-3-methoxyphenyl)but-3-en-2-one and zingerone.

The process according to the invention comprises the implementation of a step of growth of a genetically modified strain of *Pseudomonas putida* as defined above in a culture medium under conditions allowing the expression of the recombinant gene encoding the benzalacetone synthase.

Preferably, the culture conditions are the culture conditions conventionally used in a fermenter for growth of *Pseudomonas putida.*

In a particular embodiment, the method according to the present disclosure is characterized in that the culture medium comprises p-coumaroyl-coA.

In another embodiment, the process according to the present disclosure comprises carrying out a step of growing a genetically modified strain of *Pseudomonas putida* as defined above in a culture medium under conditions that allow for the expression of the recombinant gene encoding benzalacetone synthase and one or several additional recombinant gene(s) necessary to synthesize raspberry ketone or zingerone starting from L-Tyrosine or coumarate or p-coumaroyl-coA or ferulic acid.

According to a particular embodiment, the process comprises a step of growing a genetically modified strain of *Pseudomonas putida* comprising a recombinant gene encoding a benzalacetone synthase according to the invention and comprising the one, two or the three following additional recombinant genes encoding:
- a tyrosine ammonia lyase (TAL), and/or
- a 4-coumarate-CoA ligase (4-CL), and/or
- a benzalacetone reductase (BAR).

According to a particular embodiment, the synthesis process according to the invention allows the production of raspberry ketone or zingerone in large quantities, for example in yields of at least 100 mg/L, preferably at least 500 mg/L, more preferably at least 1 g/L, 1.5 g/L or 2 g/L and in particular in 2-fold, 3-fold, 4-fold, 5-fold or 6-fold production quantities in comparison with a wild-type *Pseudomonas putida* strain or one comprising the following additional recombinant genes encoding :
- a tyrosine ammonia lyase (TAL),
- a 4-coumarate-CoA ligase (4-CL),
- a benzalacetone redcutase (BAR).

In one embodiment, the process converts at least 50%, preferably 60%, 70%, 80%, 90%, 95%, or even at least 99% of the p-coumaroyl-coA synthesized in *situ* by the strain or added within the culture medium of the strain into 4-hydroxybenzalcetone.

The synthesis process according to the invention may also comprise a step of purification and/or recovery of the 4-hydroxybenzalcetone and/or raspberry ketone and/or of 4-(4-Hydroxy-3-methoxyphenyl)but-3-en-2-one and/or zingerone product by the strain.

### Examples

### Example 1: In silico study

In the literature it has been shown that the stability of an enzyme can be correlated with its catalytic efficiency. Based on this principle, (Goldenzweig, A., et al. 2016) present a protocol that estimates the free energy of an enzyme and suggests mutations to improve its stability/efficiency. An adapted version of this protocol was used to identify stabilizing mutations in the *Rheum palmatum* BAS enzyme. The protocol can be organized into the two following steps:

In the first step (1), a sequence alignment with homologous BAS proteins ahs bene performed. Rather than selecting the most promising mutations for each position, this step is performed to eliminate rare or unobserved mutations. A position specific scoring matrix is calculated and mutations with a positive score were selected. The second step (2) involves the use of the ROSETTA software to evaluate potentially stabilizing point mutations, selecting only those that result in a decrease in energy compared to the wild-type strain. An enzyme "design" ha sbeen performed where at each iteration an amino acid is mutated and the energy difference is calculated. Mutations associated with a negative energy variation were kept. Before or after the second step, unreasonable mutations can be removed. One reason for elimination is for example residues with hydrophobic chains exposed to solvent or proline introduced inside the alpha helix (Rigoldi, F., et al. 2018) or sites known to be active or responsible for a desired activity.

The first step returned a list of 490 common mutations based on the natural diversity explored. Then the second step gave us 50 residues to modify and between 1 and 3 possible mutations per residue, totaling 73 individual mutations.

Among these mutations one on residue 249 is known in the literature and is indicated on Uniprot as a mutation that reduces the activity of benzalacetone synthase. Another residue already listed by Uniprot is residue 331. However, the mutation found by our protocol is S331G while Uniprot suggests S331V as a mutation that increases benzalacetone synthase activity.

The top 20 individual mutations according to energy score were selected at the end of the study. These modified BAS are shown in the following table (the more negative the score, the more stabilizing the mutation). It should be noted that any mutated BAS disclosed in the following table may be part of the present invention, depending on its catalytic activity on the transformation of 4-coumaroyl-CoA into 4-hydroxybenzalacetone, as compared to the wild-type protein from which it is issued.

**Table 1: List of modified BAS as compared to wild-type BAS from R. palmatum and calculated energy score.**

| MUTATION on the BAS as compared to wild-type BAS from *R. palmatum* | (Energy score) ΔΔG |
|---|---|
| S257I | -1,63398 |
| S254V | -1,6365 |
| G209S | -1,78358 |
| H244E | -1,80383 |
| M186V | -1,82461 |
| Y261K | -1,89303 |
| S331G | -2,22451 |
| G209A | -2,24981 |
| T189V | -2,25927 |
| T263D | -2,36098 |
| K314P | -2,63832 |
| M186N | -2,67878 |
| Y261S | -2,98609 |
| T189I | -3,00967 |
| G249A | -3,31836 |
| S269A | -3,43104 |
| G204A | -3,87595 |
| G249L | -4,66995 |
| P30A | -5,10911 |
| T281A | -8,52003 |

BAS mutants S331G, S331V, G204A, G249L, P30A and T281A were chosen for the next step of *in vivo* testing and to verify the activity of the corresponding mutated BAS, as well as the S331V mutation as a control because it is already known in the literature.

### Example 2: In vivo study of raspberry production

### Materials and methods

### 1- Mutagenesis of the gene coding for the BAS enzyme of Rheum palmatum;

The gene coding for the BAS enzyme is cloned into the plasmid pBBR1-MCS2 (Kovach et al., 1995) downstream of the ptrc promoter which allows the overexpression of the gene in Pseudomonas putida KT2440.

Directed mutagenesis of the gene is performed with PCR primers containing the desired mutation.

Cloning and site-directed mutagenesis are performed in *E*. *coli* S17.1 strain.

### 2- Bacterial transformation

The expression plasmids of the wild type BAS (plasmid pC2F004), and of the different mutated BAS are transformed in the bacteria *Pseudomonas putida* KT2440 ppu_310 (strain which has all the enzymes for the production of raspberry ketone starting from p-coumaric acid). The strain genotype is referenced ΔPP_3358 ΔphaA::P14g_RBS007_Red_Ca ΔpykA::P14g_RBS007_4-Cl_At.

### 3- Raspberry ketone production

The different modified strains of *P*. *putida* constructed are cultured in 10mL of MPpu medium + 5g/L Glucose + p-coumaric acid (0.5mM) with seeding at OD₆₀₀ = 0.05. The cultures are placed at 30°C, under agitation 140 rpm for 48h. At the end of 48h, the cultures are centrifuged, the supernatants are recovered for HPLC assay.

### 4- HPLC assay

The concentration of molecules of p-coumaric acid (PCA), benzalacetone (HBA) and raspberry ketone (FBO) present in the supernatant are determined by HPLC. The sample is run in HPLC with Kinetex 5 µm F5 100Å column (Phenomenex) with 0.1% formic acid with acetonitrile gradient as mobile phase. The analysis method lasts 35min. The oven temperature is 40°C and the pump flow rate is 1mL/min. Detector used: UV (DAD) 280 nm, UV (DAD) 315 nm. Injection volume: 10µL. The analysis is run according to the following gradient:

**Table 2: gradient of the analysis method**

| Time (min) | % acetonitrile |
|---|---|
| 0 | 0 |
| 5 | 0 |
| 25 | 25 |
| 30 | 38 |
| 35 | 0 |

### results

As illustrated on figure 1, left panel, the concentration of pCA (4-coumaroyl-CoA) is very low in cultured *P. putida* transformed with a BAS having the T281A mutation. The pCA in these *P. putida* cells is almost completely absent. This means that pCa has been consumed by strains of P. Putida transformed with the BAS T281A. In other medium containing strains transformed with other BAS, pCA may be found in large quantities. The BAS T281A enzyme is thus more efficient than other to catalyze the transformation of pCA.

On figure 1, middle and right panels, are illustrated the concentrations of HBA (4-hydroxybenzalacetone) and FBO (raspberry ketone), obtained from the transformation of 4-coumaroyl-CoA (pCA). As illustrated, the cultured *P. putida* transformed with a BAS having the T281A mutation lead to the production of large amount of HBA and FBO, illustrating their higher capability for transforming pCA into HBA and then FBO.

As illustrated on Figure 1, the mutated BAS T281A allows *Pseudomonas putida* to consume almost all of the PCA and produce up to 0.04 mM of HBA and 0.09 mM of raspberry ketone, way over the yield obtained with other strains. Indeed, figure 2 shows that more than 115 µM of FBO raspberry ketone are obtained at the end of the process with the *P. putida* strains with the BAS T281A, while the control P. putida strains expressing wild-type BAS produce around 20 µM of raspberry ketone. The *P. putida* strains with the BAS T281A are at least 6 times more efficient than prior art strains for transforming pCA into HBA, and *in fine* for providing raspberry ketone. These results clearly illustrate the higher efficiency of *P. putida* strains transformed with the BAS T281A for producing HBA and *in fine* FBO as compared to P. putida strain transformed with wild-type BAS or with BAS presenting other mutation than T281A. The mutated BAS T281A allows *Pseudomonas putida* to consume almost all of the PCA and produce up to 0.04 mM of HBA and 0.09 mM of raspberry ketone, way over the yield obtained with other strains.

### Example 3: In vivo study of zingerone production

### Materials and methods

Mutagenesis, Bacterial transformation and HPLC assays were performed according to example 2. For HPLC, the concentration of molecules of ferulic acid (Fer), vanillylidene acetone (Vda), and zingerone (ZGO) present in the supernatant are determined.

The zingerone production is determined in presence of different strains of *P. putida* cultured in 10mL of MPpu medium + 5g/L Glucose + 1mM ferulic acid with seeding at OD₆₀₀ = 0.05. The cultures are placed at 30°C, under agitation 140 rpm for 48h. At the end of 48h, the cultures are centrifuged, the supernatants are recovered for HPLC assay.

### results

As illustrated on figure 3, left panel, the concentration of ferulic acid (Fer) is very low in cultured *P. putida* transformed with a BAS having the T281A mutation. The Fer in these *P. putida* cells is almost completely absent and has been consumed by the transformed *P. Putida.* On the contrary, Fer may be found in the medium where strains of untransformed *P. putida* or *P. putida* transformed with wild-type BAS. The BAS T281A enzyme seems thus more efficient than other to catalyze the transformation of Fer.

On figure 3, middle and right panels, are illustrated the concentrations of vanillylidene acetone (Vda) and ZGO (zingerone), obtained from the transformation of ferulic acid. As illustrated, the cultured *P. putida* transformed with a BAS having the T281A mutation lead to the production of large amount of Vda and ZGO, illustrating their higher capability for transforming pCA into HBA and then FBO.

As illustrated on Figure 3, the mutated BAS T281A allows *Pseudomonas putida* to consume almost all of the Fer and produce up to 0.06 mM of Fer and 0.04 mM of zingerone, way over the yield obtained with other strains. The *P. putida* strains with the BAS T281A are more efficient than prior art strains for transforming Fer into Vda, and *in fine* for providing zingerone.

These results clearly illustrate the higher efficiency of *P. putida* strains transformed with the BAS T281A for producing Vda and ZGO as compared to P. putida strain transformed with wild-type BAS. The mutated BAS T281A allows *Pseudomonas putida* to consume almost all of the Fer and produce up to 0.06 mM of Vda and 0.04 mM of zingeroe, way over the yield obtained with other strains.

### Sequence listing

| Name | Sequences |
|---|---|
| Bas_Wt | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81A | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFAPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81A gene | ATGGCAACTGAGGAGATGAAGAAATTGGCCACCGTGATGGCCATTGGCACGGC CAACCCTCCGAACTGCTACTACCAGGCCGACTTTCCCGACTTCTACTTCCGCGT CACCAACAGCGACCACCTCATCAACCTCAAGCAAAAGTTCAAGCGCCTTTGTGA AAACTCAAGGATTGAGAAGCGTTACCTTCATGTGACCGAAGAGATTCTCAAGGA AAACCCAAACATTGCTGCCTACGAGGCAACCTCGTTGAATGTAAGACACAAAAT GCAAGTGAAAGGAGTTGCAGAGCTTGGGAAAGAGGCTGCCCTCAAGGCCATCA AAGAATGGGGCCAACCCAAGTCCAAGATCACACATCTCATCGTGTGTTGCCTAG CCGGCGTTGACATGCCCGGCGCGGATTATCAACTCACTAAGCTTCTTGACCTTG |
| | ACCCTTCCGTCAAGCGTTTTATGTTTTACCACCTAGGATGCTACGCTGGTGGCAC TGTCCTTCGCCTTGCAAAGGACATAGCGGAGAACAACAAGGGAGCTCGTGTTCT CATCGTTTGCTCAGAGATGACAACAACTTGTTTTCGTGGGCCATCTGAAACCCAT CTGGACTCCATGATAGGCCAAGCAATATTAGGCGATGGGGCTGCAGCTGTCATA GTTGGCGCAGATCCAGACCTAACCGTTGAGAGGCCCATATTCGAGTTGGTTTCC ACAGCCCAGACTATTGTACCCGAATCCCATGGTGCAATTGAGGGCCACTTGCTT GAATCTGGACTCAGTTTCCATTTGTACAAGACCGTTCCTACACTAATCTCTAACA ACATTAAAACTTGCCTTTCTGATGCTTTCGCTCCTCTAAACATTAGCGATTGGAAC TCTCTTTTCTGGATCGCACACCCTGGTGGTCCTGCCATCCTAGACCAAGTTACTG CTAAGGTTGGTCTTGAAAAGGAGAAACTCAAGGTAACTAGACAAGTGTTGAAGG ACTATGGAAACATGTCGAGTGCTACGGTGTTTTTCATCATGGATGAGATGAGGAA GAAGTCACTCGAAAACGGTCAAGCAACCACTGGAGAAGGGCTCGAGTGGGGTG TTTTGTTTGGGTTCGGGCCTGGAATCACCGTTGAAACTGTAGTGCTACGCAGTG TGCCCGTAATTAGCTAG |
| pBBR1 Ptrc_Ba s_T281 A plasmid and gene | CTCGGGCCGTCTCTTGGGCTTGATCGGCCTTCTTGCGCATCTCACGCGCTCCTG CGGCGGCCTGTAGGGCAGGCTCATACCCCTGCCGAACCGCTTTTGTCAGCCGG TCGGCCACGGCTTCCGGCGTCTCAACGCGCTTTGAGATTCCCAGCTTTTCGGCC AATCCCTGCGGTGCATAGGCGCGTGGCTCGACCGCTTGCGGGCTGATGGTGAC GTGGCCCACTGGTGGCCGCTCCAGGGCCTCGTAGAACGCCTGAATGCGCGTGT GACGTGCCTTGCTGCCCTCGATGCCCCGTTGCAGCCCTAGATCGGCCACAGCG GCCGCAAACGTGGTCTGGTCGCGGGTCATCTGCGCTTTGTTGCCGATGAACTC CTTGGCCGACAGCCTGCCGTCCTGCGTCAGCGGCACCACGAACGCGGTCATGT GCGGGCTGGTTTCGTCACGGTGGATGCTGGCCGTCACGATGCGATCCGCCCCG TACTTGTCCGCCAGCCACTTGTGCGCCTTCTCGAAGAACGCCGCCTGCTGTTCT TGGCTGGCCGACTTCCACCATTCCGGGCTGGCCGTCATGACGTACTCGACCGC CAACACAGCGTCCTTGCGCCGCTTCTCTGGCAGCAACTCGCGCAGTCGGCCCA TCGCTTCATCGGTGCTGCTGGCCGCCCAGTGCTCGTTCTCTGGCGTCCTGCTG GCGTCAGCGTTGGGCGTCTCGCGCTCGCGGTAGGCGTGCTTGAGACTGGCCG CCACGTTGCCCATTTTCGCCAGCTTCTTGCATCGCATGATCGCGTATGCCGCCA TGCCTGCCCCTCCCTTTTGGTGTCCAACCGGCTCGACGGGGGCAGCGCAAGGC GGTGCCTCCGGCGGGCCACTCAATGCTTGAGTATACTCACTAGACTTTGCTTCG CAAAGTCGTGACCGCCTACGGCGGCTGCGGCGCCCTACGGGCTTGCTCTCCGG GCTTCGCCCTGCGCGGTCGCTGCGCTCCCTTGCCAGCCCGTGGATATGTGGAC GATGGCCGCGAGCGGCCACCGGCTGGCTCGCTTCGCTCGGCCCGTGGACAAC CCTGCTGGACAAGCTGATGGACAGGCTGCGCCTGCCCACGAGCTTGACCACAG GGATTGCCCACCGGCTACCCAGCCTTCGACCACATACCCACCGGCTCCAACTG CGCGGCCTGCGGCCTTGCCCCATCAATTTTTTTAATTTTCTCTGGGGAAAAGCCT CCGGCCTGCGGCCTGCGCGCTTCGCTTGCCGGTTGGACACCAAGTGGAAGGC |
| | GGGTCAAGGCTCGCGCAGCGACCGCGCAGCGGCTTGGCCTTGACGCGCCTGG AACGACCCAAGCCTATGCGAGTGGGGGCAGTCGAAGGCGAAGCCCGCCCGCC TGCCCCCCGAGCCTCACGGCGGCGAGTGCGGGGGTTCCAAGGGGGCAGCGCC ACCTTGGGCAAGGCCGAAGGCCGCGCAGTCGATCAACAAGCCCCGGAGGGGC CACTTTTTGCCGGAGGGGGAGCCGCGCCGAAGGCGTGGGGGAACCCCGCAGG GGTGCCCTTCTTTGGGCACCAAAGAACTAGATATAGGGCGAAATGCGAAAGACT TAAAAATCAACAACTTAAAAAAGGGGGGTACGCAACAGCTCATTGCGGCACCCC CCGCAATAGCTCATTGCGTAGGTTAAAGAAAATCTGTAATTGACTGCCACTTTTA CGCAACGCATAATTGTTGTCGCGCTGCCGAAAAGTTGCAGCTGATTGCGCATGG TGCCGCAACCGTGCGGCACCCTACCGCATGGAGATAAGCATGGCCACGCAGTC CAGAGAAATCGGCATTCAAGCCAAGAACAAGCCCGGTCACTGGGTGCAAACGG AACGCAAAGCGCATGAGGCGTGGGCCGGGCTTATTGCGAGGAAACCCACGGC GGCAATGCTGCTGCATCACCTCGTGGCGCAGATGGGCCACCAGAACGCCGTGG TGGTCAGCCAGAAGACACTTTCCAAGCTCATCGGACGTTCTTTGCGGACGGTCC AATACGCAGTCAAGGACTTGGTGGCCGAGCGCTGGATCTCCGTCGTGAAGCTC AACGGCCCCGGCACCGTGTCGGCCTACGTGGTCAATGACCGCGTGGCGTGGG GCCAGCCCCGCGACCAGTTGCGCCTGTCGGTGTTCAGTGCCGCCGTGGTGGTT GATCACGACGACCAGGACGAATCGCTGTTGGGGCATGGCGACCTGCGCCGCAT CCCGACCCTGTATCCGGGCGAGCAGCAACTACCGACCGGCCCCGGCGAGGAG CCGCCCAGCCAGCCCGGCATTCCGGGCATGGAACCAGACCTGCCAGCCTTGAC CGAAACGGAGGAATGGGAACGGCGCGGGCAGCAGCGCCTGCCGATGCCCGAT GAGCCGTGTTTTCTGGACGATGGCGAGCCGTTGGAGCCGCCGACACGGGTCAC GCTGCCGCGCCGGTAGGGCATCAAATAAAACGAAAGGCTCAGTCGAAAGACTG GGCCTTTCGTTTTATCTGTTGTTTGTCGGTGAACGCTCTCCTGAGTAGGACAAAT CCGCCGCCCTAGAGCGCAACGCAATTAATGTAAGTTAGCGCGAATTGATCTGGT TTGACAGCTTATCATCGACTGCACGGTGCACCAATGCTTCTGGCGTCAGGCAGC CATCGGAAGCTGTGGTATGGCTGTGCAGGTCGTAAATCACTGCATAATTCGTGT CGCTCAAGGCGCACTCCCGTTCTGGATAATGTTTTTTGCGCCGACATCATAACG GTTCTGGCAAATATTCTGAAATGAGCTGTTGACAATTAATCATCCGGCTCGTATA ATGTGTGGAATTGTGAGCGGATAACAATTAGGAGGAAAAACATATGGCAACTGA GGAGATGAAGAAATTGGCCACCGTGATGGCCATTGGCACGGCCAACCCTCCGA ACTGCTACTACCAGGCCGACTTTCCCGACTTCTACTTCCGCGTCACCAACAGCG ACCACCTCATCAACCTCAAGCAAAAGTTCAAGCGCCTTTGTGAAAACTCAAGGAT TGAGAAGCGTTACCTTCATGTGACCGAAGAGATTCTCAAGGAAAACCCAAACATT GCTGCCTACGAGGCAACCTCGTTGAATGTAAGACACAAAATGCAAGTGAAAGGA GTTGCAGAGCTTGGGAAAGAGGCTGCCCTCAAGGCCATCAAAGAATGGGGCCA ACCCAAGTCCAAGATCACACATCTCATCGTGTGTTGCCTAGCCGGCGTTGACAT GCCCGGCGCGGATTATCAACTCACTAAGCTTCTTGACCTTGACCCTTCCGTCAA |
| | GCGTTTTATGTTTTACCACCTAGGATGCTACGCTGGTGGCACTGTCCTTCGCCTT GCAAAGGACATAGCGGAGAACAACAAGGGAGCTCGTGTTCTCATCGTTTGCTCA GAGATGACAACAACTTGTTTTCGTGGGCCATCTGAAACCCATCTGGACTCCATG ATAGGCCAAGCAATATTAGGCGATGGGGCTGCAGCTGTCATAGTTGGCGCAGAT CCAGACCTAACCGTTGAGAGGCCCATATTCGAGTTGGTTTCCACAGCCCAGACT ATTGTACCCGAATCCCATGGTGCAATTGAGGGCCACTTGCTTGAATCTGGACTC AGTTTCCATTTGTACAAGACCGTTCCTACACTAATCTCTAACAACATTAAAACTTG CCTTTCTGATGCTTTCGCTCCTCTAAACATTAGCGATTGGAACTCTCTTTTCTGGA TCGCACACCCTGGTGGTCCTGCCATCCTAGACCAAGTTACTGCTAAGGTTGGTC TTGAAAAGGAGAAACTCAAGGTAACTAGACAAGTGTTGAAGGACTATGGAAACAT GTCGAGTGCTACGGTGTTTTTCATCATGGATGAGATGAGGAAGAAGTCACTCGA AAACGGTCAAGCAACCACTGGAGAAGGGCTCGAGTGGGGTGTTTTGTTTGGGTT CGGGCCTGGAATCACCGTTGAAACTGTAGTGCTACGCAGTGTGCCCGTAATTAG CTAGCTTGGACTCCTGTTGATAGATCCAGTAATGACCTCAGAACTCCATCTGGAT TTGTTCAGAACGCTCGGTTGCCGCCGGGCGTTTTTTATTGGTGAGAATCCAGAT TGCGTTGCGCTCACTGCCCGCTTTCCAGTCGGGAAACCTGTCGTGCCAGCTGC ATTAATGAATCGGCCAACGCGCGGGGAGAGGCGGTTTGCGTATTGGGCGCATG CATAAAAACTGTTGTAATTCATTAAGCATTCTGCCGACATGGAAGCCATCACAAA CGGCATGATGAACCTGAATCGCCAGCGGCATCAGCACCTTGTCGCCTTGCGTAT AATATTTGCCCATGGGGGTGGGCGAAGAACTCCAGCATGAGATCCCCGCGCTG GAGGATCATCCAGCCGGCGTCCCGGAAAACGATTCCGAAGCCCAACCTTTCATA GAAGGCGGCGGTGGAATCGAAATCTCGTGATGGCAGGTTGGGCGTCGCTTGGT CGGTCATTTCGAACCCCAGAGTCCCGCTCAGAAGAACTCGTCAAGAAGGCGATA GAAGGCGATGCGCTGCGAATCGGGAGCGGCGATACCGTAAAGCACGAGGAAG CGGTCAGCCCATTCGCCGCCAAGCTCTTCAGCAATATCACGGGTAGCCAACGCT ATGTCCTGATAGCGGTCCGCCACACCCAGCCGGCCACAGTCGATGAATCCAGA AAAGCGGCCATTTTCCACCATGATATTCGGCAAGCAGGCATCGCCATGGGTCAC GACGAGATCCTCGCCGTCGGGCATGCGCGCCTTGAGCCTGGCGAACAGTTCGG CTGGCGCGAGCCCCTGATGCTCTTCGTCCAGATCATCCTGATCGACAAGACCG GCTTCCATCCGAGTACGTGCTCGCTCGATGCGATGTTTCGCTTGGTGGTCGAAT GGGCAGGTAGCCGGATCAAGCGTATGCAGCCGCCGCATTGCATCAGCCATGAT GGATACTTTCTCGGCAGGAGCAAGGTGAGATGACAGGAGATCCTGCCCCGGCA CTTCGCCCAATAGCAGCCAGTCCCTTCCCGCTTCAGTGACAACGTCGAGCACAG CTGCGCAAGGAACGCCCGTCGTGGCCAGCCACGATAGCCGCGCTGCCTCGTCC TGCAGTTCATTCAGGGCACCGGACAGGTCGGTCTTGACAAAAAGAACCGGGCG CCCCTGCGCTGACAGCCGGAACACGGCGGCATCAGAGCAGCCGATTGTCTGTT GTGCCCAGTCATAGCCGAATAGCCTCTCCACCCAAGCGGCCGGAGAACCTGCG TGCAATCCATCTTGTTCAATCATGCGAAACGATCCTCATCCTGTCTCTTGATCAG |
| | ATCTTGATCCCCTGCGCCATCAGATCCTTGGCGGCAAGAAAGCCATCCAGTTTA CTTTGCAGGGCTTCCCAACCTTACCAGAGGGCGCCCCAGCTGGCAATTCCGGTT CGCTTGCTGTCCATAAAACCGCCCAGTCTAGCTATCGCCATGTAAGCCCACTGC AAGCTACCTGCTTTCTCTTTGCGCTTGCGTTTTCCCTTGTCCAGATAGCCCAGTA GCTGACATTCATCCCAGGTGGCACTTTTCGGGGAAATGTGCGCGCCCGCGTTC CTGCTGGCGCTGGGCCTGTTTCTGGCGCTGGACTTCCCGCTGTTCCGTCAGCA GCTTTTCGCCCACGGCCTTGATGATCGCGGCGGCCTTGGCCTGCATATCCCGAT TCAACGGCCCCAGGGCGTCCAGAACGGGCTTCAGGCGCTCCCGAAGGT |
| Bas_W T gene | ATGGCAACTGAGGAGATGAAGAAATTGGCCACCGTGATGGCCATTGGCACGGC CAACCCTCCGAACTGCTACTACCAGGCCGACTTTCCCGACTTCTACTTCCGCGT CACCAACAGCGACCACCTCATCAACCTCAAGCAAAAGTTCAAGCGCCTTTGTGA AAACTCAAGGATTGAGAAGCGTTACCTTCATGTGACCGAAGAGATTCTCAAGGA AAACCCAAACATTGCTGCCTACGAGGCAACCTCGTTGAATGTAAGACACAAAAT GCAAGTGAAAGGAGTTGCAGAGCTTGGGAAAGAGGCTGCCCTCAAGGCCATCA AAGAATGGGGCCAACCCAAGTCCAAGATCACACATCTCATCGTGTGTTGCCTAG CCGGCGTTGACATGCCCGGCGCGGATTATCAACTCACTAAGCTTCTTGACCTTG ACCCTTCCGTCAAGCGTTTTATGTTTTACCACCTAGGATGCTACGCTGGTGGCAC TGTCCTTCGCCTTGCAAAGGACATAGCGGAGAACAACAAGGGAGCTCGTGTTCT CATCGTTTGCTCAGAGATGACAACAACTTGTTTTCGTGGGCCATCTGAAACCCAT CTGGACTCCATGATAGGCCAAGCAATATTAGGCGATGGGGCTGCAGCTGTCATA GTTGGCGCAGATCCAGACCTAACCGTTGAGAGGCCCATATTCGAGTTGGTTTCC ACAGCCCAGACTATTGTACCCGAATCCCATGGTGCAATTGAGGGCCACTTGCTT GAATCTGGACTCAGTTTCCATTTGTACAAGACCGTTCCTACACTAATCTCTAACA ACATTAAAACTTGCCTTTCTGATGCTTTCACTCCTCTAAACATTAGCGATTGGAAC TCTCTTTTCTGGATCGCACACCCTGGTGGTCCTGCCATCCTAGACCAAGTTACTG CTAAGGTTGGTCTTGAAAAGGAGAAACTCAAGGTAACTAGACAAGTGTTGAAGG ACTATGGAAACATGTCGAGTGCTACGGTGTTTTTCATCATGGATGAGATGAGGAA GAAGTCACTCGAAAACGGTCAAGCAACCACTGGAGAAGGGCTCGAGTGGGGTG TTTTGTTTGGGTTCGGGCCTGGAATCACCGTTGAAACTGTAGTGCTACGCAGTG TGCCCGTAATTAGCTAG |
| pBBR1 plasmid | CTCGGGCCGTCTCTTGGGCTTGATCGGCCTTCTTGCGCATCTCACGCGCTCCTG CGGCGGCCTGTAGGGCAGGCTCATACCCCTGCCGAACCGCTTTTGTCAGCCGG TCGGCCACGGCTTCCGGCGTCTCAACGCGCTTTGAGATTCCCAGCTTTTCGGCC AATCCCTGCGGTGCATAGGCGCGTGGCTCGACCGCTTGCGGGCTGATGGTGAC GTGGCCCACTGGTGGCCGCTCCAGGGCCTCGTAGAACGCCTGAATGCGCGTGT GACGTGCCTTGCTGCCCTCGATGCCCCGTTGCAGCCCTAGATCGGCCACAGCG GCCGCAAACGTGGTCTGGTCGCGGGTCATCTGCGCTTTGTTGCCGATGAACTC |
| | CTTGGCCGACAGCCTGCCGTCCTGCGTCAGCGGCACCACGAACGCGGTCATGT GCGGGCTGGTTTCGTCACGGTGGATGCTGGCCGTCACGATGCGATCCGCCCCG TACTTGTCCGCCAGCCACTTGTGCGCCTTCTCGAAGAACGCCGCCTGCTGTTCT TGGCTGGCCGACTTCCACCATTCCGGGCTGGCCGTCATGACGTACTCGACCGC CAACACAGCGTCCTTGCGCCGCTTCTCTGGCAGCAACTCGCGCAGTCGGCCCA TCGCTTCATCGGTGCTGCTGGCCGCCCAGTGCTCGTTCTCTGGCGTCCTGCTG GCGTCAGCGTTGGGCGTCTCGCGCTCGCGGTAGGCGTGCTTGAGACTGGCCG CCACGTTGCCCATTTTCGCCAGCTTCTTGCATCGCATGATCGCGTATGCCGCCA TGCCTGCCCCTCCCTTTTGGTGTCCAACCGGCTCGACGGGGGCAGCGCAAGGC GGTGCCTCCGGCGGGCCACTCAATGCTTGAGTATACTCACTAGACTTTGCTTCG CAAAGTCGTGACCGCCTACGGCGGCTGCGGCGCCCTACGGGCTTGCTCTCCGG GCTTCGCCCTGCGCGGTCGCTGCGCTCCCTTGCCAGCCCGTGGATATGTGGAC GATGGCCGCGAGCGGCCACCGGCTGGCTCGCTTCGCTCGGCCCGTGGACAAC CCTGCTGGACAAGCTGATGGACAGGCTGCGCCTGCCCACGAGCTTGACCACAG GGATTGCCCACCGGCTACCCAGCCTTCGACCACATACCCACCGGCTCCAACTG CGCGGCCTGCGGCCTTGCCCCATCAATTTTTTTAATTTTCTCTGGGGAAAAGCCT CCGGCCTGCGGCCTGCGCGCTTCGCTTGCCGGTTGGACACCAAGTGGAAGGC GGGTCAAGGCTCGCGCAGCGACCGCGCAGCGGCTTGGCCTTGACGCGCCTGG AACGACCCAAGCCTATGCGAGTGGGGGCAGTCGAAGGCGAAGCCCGCCCGCC TGCCCCCCGAGCCTCACGGCGGCGAGTGCGGGGGTTCCAAGGGGGCAGCGCC ACCTTGGGCAAGGCCGAAGGCCGCGCAGTCGATCAACAAGCCCCGGAGGGGC CACTTTTTGCCGGAGGGGGAGCCGCGCCGAAGGCGTGGGGGAACCCCGCAGG GGTGCCCTTCTTTGGGCACCAAAGAACTAGATATAGGGCGAAATGCGAAAGACT TAAAAATCAACAACTTAAAAAAGGGGGGTACGCAACAGCTCATTGCGGCACCCC CCGCAATAGCTCATTGCGTAGGTTAAAGAAAATCTGTAATTGACTGCCACTTTTA CGCAACGCATAATTGTTGTCGCGCTGCCGAAAAGTTGCAGCTGATTGCGCATGG TGCCGCAACCGTGCGGCACCCTACCGCATGGAGATAAGCATGGCCACGCAGTC CAGAGAAATCGGCATTCAAGCCAAGAACAAGCCCGGTCACTGGGTGCAAACGG AACGCAAAGCGCATGAGGCGTGGGCCGGGCTTATTGCGAGGAAACCCACGGC GGCAATGCTGCTGCATCACCTCGTGGCGCAGATGGGCCACCAGAACGCCGTGG TGGTCAGCCAGAAGACACTTTCCAAGCTCATCGGACGTTCTTTGCGGACGGTCC AATACGCAGTCAAGGACTTGGTGGCCGAGCGCTGGATCTCCGTCGTGAAGCTC AACGGCCCCGGCACCGTGTCGGCCTACGTGGTCAATGACCGCGTGGCGTGGG GCCAGCCCCGCGACCAGTTGCGCCTGTCGGTGTTCAGTGCCGCCGTGGTGGTT GATCACGACGACCAGGACGAATCGCTGTTGGGGCATGGCGACCTGCGCCGCAT CCCGACCCTGTATCCGGGCGAGCAGCAACTACCGACCGGCCCCGGCGAGGAG CCGCCCAGCCAGCCCGGCATTCCGGGCATGGAACCAGACCTGCCAGCCTTGAC CGAAACGGAGGAATGGGAACGGCGCGGGCAGCAGCGCCTGCCGATGCCCGAT |
| | GAGCCGTGTTTTCTGGACGATGGCGAGCCGTTGGAGCCGCCGACACGGGTCAC GCTGCCGCGCCGGTAGGGCATCAAATAAAACGAAAGGCTCAGTCGAAAGACTG GGCCTTTCGTTTTATCTGTTGTTTGTCGGTGAACGCTCTCCTGAGTAGGACAAAT CCGCCGCCCTAGAGCGCAACGCAATTAATGTAAGTTAGCGCGAATTGATCTGGT TTGACAGCTTATCATCGACTGCACGGTGCACCAATGCTTCTGGCGTCAGGCAGC CATCGGAAGCTGTGGTATGGCTGTGCAGGTCGTAAATCACTGCATAATTCGTGT CGCTCAAGGCGCACTCCCGTTCTGGATAATGTTTTTTGCGCCGACATCATAACG GTTCTGGCAAATATTCTGAAATGAGCTGTTGACAATTAATCATCCGGCTCGTATA ATGTGTGGAATTGTGAGCGGATAACAATTAGGAGGAAAAACATCTTGGACTCCT GTTGATAGATCCAGTAATGACCTCAGAACTCCATCTGGATTTGTTCAGAACGCTC GGTTGCCGCCGGGCGTTTTTTATTGGTGAGAATCCAGATTGCGTTGCGCTCACT GCCCGCTTTCCAGTCGGGAAACCTGTCGTGCCAGCTGCATTAATGAATCGGCCA ACGCGCGGGGAGAGGCGGTTTGCGTATTGGGCGCATGCATAAAAACTGTTGTA ATTCATTAAGCATTCTGCCGACATGGAAGCCATCACAAACGGCATGATGAACCT GAATCGCCAGCGGCATCAGCACCTTGTCGCCTTGCGTATAATATTTGCCCATGG GGGTGGGCGAAGAACTCCAGCATGAGATCCCCGCGCTGGAGGATCATCCAGCC GGCGTCCCGGAAAACGATTCCGAAGCCCAACCTTTCATAGAAGGCGGCGGTGG AATCGAAATCTCGTGATGGCAGGTTGGGCGTCGCTTGGTCGGTCATTTCGAACC CCAGAGTCCCGCTCAGAAGAACTCGTCAAGAAGGCGATAGAAGGCGATGCGCT GCGAATCGGGAGCGGCGATACCGTAAAGCACGAGGAAGCGGTCAGCCCATTCG CCGCCAAGCTCTTCAGCAATATCACGGGTAGCCAACGCTATGTCCTGATAGCGG TCCGCCACACCCAGCCGGCCACAGTCGATGAATCCAGAAAAGCGGCCATTTTCC ACCATGATATTCGGCAAGCAGGCATCGCCATGGGTCACGACGAGATCCTCGCC GTCGGGCATGCGCGCCTTGAGCCTGGCGAACAGTTCGGCTGGCGCGAGCCCC TGATGCTCTTCGTCCAGATCATCCTGATCGACAAGACCGGCTTCCATCCGAGTA CGTGCTCGCTCGATGCGATGTTTCGCTTGGTGGTCGAATGGGCAGGTAGCCGG ATCAAGCGTATGCAGCCGCCGCATTGCATCAGCCATGATGGATACTTTCTCGGC AGGAGCAAGGTGAGATGACAGGAGATCCTGCCCCGGCACTTCGCCCAATAGCA GCCAGTCCCTTCCCGCTTCAGTGACAACGTCGAGCACAGCTGCGCAAGGAACG CCCGTCGTGGCCAGCCACGATAGCCGCGCTGCCTCGTCCTGCAGTTCATTCAG GGCACCGGACAGGTCGGTCTTGACAAAAAGAACCGGGCGCCCCTGCGCTGACA GCCGGAACACGGCGGCATCAGAGCAGCCGATTGTCTGTTGTGCCCAGTCATAG CCGAATAGCCTCTCCACCCAAGCGGCCGGAGAACCTGCGTGCAATCCATCTTGT TCAATCATGCGAAACGATCCTCATCCTGTCTCTTGATCAGATCTTGATCCCCTGC GCCATCAGATCCTTGGCGGCAAGAAAGCCATCCAGTTTACTTTGCAGGGCTTCC CAACCTTACCAGAGGGCGCCCCAGCTGGCAATTCCGGTTCGCTTGCTGTCCATA AAACCGCCCAGTCTAGCTATCGCCATGTAAGCCCACTGCAAGCTACCTGCTTTC TCTTTGCGCTTGCGTTTTCCCTTGTCCAGATAGCCCAGTAGCTGACATTCATCCC |
| | AGGTGGCACTTTTCGGGGAAATGTGCGCGCCCGCGTTCCTGCTGGCGCTGGGC CTGTTTCTGGCGCTGGACTTCCCGCTGTTCCGTCAGCAGCTTTTCGCCCACGGC CTTGATGATCGCGGCGGCCTTGGCCTGCATATCCCGATTCAACGGCCCCAGGG CGTCCAGAACGGGCTTCAGGCGCTCCCGAAGGT |
| Bas_T2 81L | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFLPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFIPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| Bas_T2 81I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALNAIKEWGQP KSKITHLIVCCLAGVDMPGADYQLTKLLDLDPSVKRFMFYHLGCYAGGTVLRLAKDIA ENNKGARVLIVCSEMTTTCFRGPSETHLDSMIGQAILGDGAAAVIVGADPDLTVERPI FELVSTAQTIVPESHGAIEGHLLESGLSFHLYKTVPTLISNNIKTCLSDAFIPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| K105N | |
| Bas_T2 81I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWDQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFIPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| G111D | |
| Bas_T2 81C | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF |
| | ELVSTAQTIVPESHGAIEGHLLESGLSFHLYKTVPTLISNNIKTCLSDAFCPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81K | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFKPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81K | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTIPTLISNNIKTCLSDAFKPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| V264I | |
| Bas_L1 41I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLIDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T2 81A | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLIDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFAPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| L141I | |
| Bas_R5 1K | MATEEMKKLATVMAIGTANPPNCYYQADFPDFYFRVTNSDHLINLKQKFKKLCENSR IEKRYLHVTEEILKENPNIAAYEATSLNVRHKMQVKGVAELGKEAALKAIKEWGQPKS KITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN |
| | SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_N8 5D | MATEEMKKLATVMAIGTANPPNCYYQADFPDFYFRVTNSDHLINLKQKFKRLCENS RIEKRYLHVTEEILKENPNIAAYEATSLDVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_M1 86N | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSENTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_M1 86V | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEVTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T1 89C | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTCCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T1 89I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTICFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN |
| | SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_T1 89V | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTVCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_G2 09A | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILADGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_G2 09S | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILSDGAAAVIVGADPDLTVERPIF ELVSTAQTIVPESHGAIEGHLLESGLSFHLYKTVPTLISNNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_S2 69A | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLIANNIKTCLSDAFTPLNISDWN SLFWIAHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_A2 95V | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTVPTLISNNIKTCLSDAFTPLNISDWN |
| | SLFWIVHPGGPAILDQVTAKVGLEKEKLKVTRQVLKDYGNMSSATVFFIMDEMRKKS LENGQATTGEGLEWGVLFGFGPGITVETVVLRSVPVIS |
| Bas_V2 64I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLLDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTIPTLISNNIKTCLSDAFTPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| Bas_V2 64I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLIDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTIPTLISNNIKTCLSDAFTPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| L141I | |
| Bas_L1 41I | MATEEMKKLATVMAIGTANPPNCYYQADFPDFYFRVTNSDHLINLKQKFKRLCENS RIEKRYLHVTEEILKENPNIAAYEATSLNVRHKMQVKGVAELGKEAALKAIKEWGQPK SKITHLIVCCLAGVDMPGADYQLTKLIDLDPSVKRFMFYHLGCYAGGTVLRLAKDIAE NNKGARVLIVCSEMTTTCFRGPSETHLDSMIGQAILGDGAAAVIVGADPDLTVERPIF ELVSTAQTIVPESHGAIEGHLLESGLSFHLYKTIPTLISNNIKTCLSDAFAPLNISDWNS LFWIAHPGGPAILDQVTAKVGLEKEKLKVTRQVLKDYGNMSSATVFFIMDEMRKKSL ENGQATTGEGLEWGVLFGFGPGITVETVVLRSVPVIS |
| V264I | |
| T281A | |

### List of cited documents

### Patent literature

WO2001011071 - Bioproduction of para-hydroxycinnamic acid
WO2002010407 - Bioproduction of para-hydroxycinnamic acid
GB2416770 - Synthesis of benzalacetone/raspberry ketone by chalcone synthase.
GB0416830 - Biosynthesis of raspberry ketone.
WO2017207950 - Production of frambinone by a recombinant fungal microorganism.
WO2019097049 - Production of a flavour compound in a host cell.

### Non-patent literature

Abe, Ikuro, et al. « Benzalacetone Synthase: A Novel Polyketide Synthase That Plays a Crucial Role in the Biosynthesis of Phenylbutanones in Rheum Palmatum ». European Journal of Biochemistry, vol. 268, n° 11, juin 2001, p. 3354-59. DOI.org (Crossref), https://doi.org/10.1046/j.1432-1327.2001.02255.x.
Abe, Tsuyoshi, et al. « Structure Function Analysis of Benzalacetone Synthase from Rheum Palmatum ». Bioorganic & Medicinal Chemistry Letters, vol. 17, n° 11, juin 2007, p. 3161-66. ScienceDirect, https://doi.org/10.1016/j.bmcl.2007.03.029.
Chang, Chen, et al. « Efficient Bioconversion of Raspberry Ketone in Escherichia Coli Using Fatty Acids Feedstocks ». Microbial Cell Factories, vol. 20, n° 1, mars 2021, p. 68. PubMed, https://doi.org/10.1186/s12934-021-01551-0.
Goldenzweig, Adi, et al. « Automated Structure- and Sequence-Based Design of Proteins for High Bacterial Expression and Stability ». Molecular Cell, vol. 63, n° 2, juillet 2016, p. 337-46. PubMed, https://doi.org/10.1016/j.molcel.2016.06.012.
Häkkinen, Suvi T., et al. « Bioconversion to Raspberry Ketone is Achieved by Several Non-related Plant Cell Cultures ». Frontiers in Plant Science, vol. 6, novembre 2015. PubMed Central, https://doi.org/10.3389/fpls.2015.01035.
Kovach, M. E., et al. « Four New Derivatives of the Broad-Host-Range Cloning Vector PBBR1MCS, Carrying Different Antibiotic-Resistance Cassettes ». Gene, vol. 166, n° 1, décembre 1995, p. 175-76.
Lee, Danna, et al. « Heterologous production of raspberry ketone in the wine yeast Saccharomyces cerevisiae via pathway engineering and synthetic enzyme fusion ». Microbial Cell Factories, vol. 15, mars 2016. PubMed Central, https://doi.org/10.1186/s12934-016-0446-2.
Liu, Chang, et Sijin Li. « Engineered Biosynthesis of Plant Polyketides by Type III Polyketide Synthases in Microorganisms ». Frontiers in Bioengineering and Biotechnology, vol. 10, 2022, p. 1017190. PubMed, https://doi.org/10.3389/fbioe.2022.1017190.
Milke, Lars, et al. « Synthesis of the Character Impact Compound Raspberry Ketone and Additional Flavoring Phenylbutanoids of Biotechnological Interest with Corynebacterium Glutamicum ». Microbial Cell Factories, vol. 19, n° 1, avril 2020, p. 92. PubMed, https://doi.org/10.1186/s12934-020-01351-y.
Morita, H., et al. « A Structure-Based Mechanism for Benzalacetone Synthase from Rheum Palmatum. » Proceedings of the National Academy of Sciences of the United States of America, vol. 107, n° 2, janvier 2010, p. 669-73. europepmc.org, https://doi.org/10.1073/pnas.0909982107.
Rigoldi, Federica, et al. « Review: Engineering of Thermostable Enzymes for Industrial Applications ». APL Bioengineering, vol. 2, n° 1, mars 2018, p. 011501. PubMed, https://doi.org/10.1063/1.4997367.
Shimokawa, Yoshihiko, et al. « Benzalacetone Synthase ». Frontiers in plant science, vol. 3, mars 2012. PubMed Central, https://doi.org/10.3389/fpls.2012.00057.
Wang, Chengcheng, et al. « Construction of Synthetic Pathways for Raspberry Ketone Production in Engineered Escherichia Coli ». Applied Microbiology and Biotechnology, mars 2019. Crossref, https://doi.org/10.1007/s00253-019-09748-5.

## Claims

1. A modified benzalacetone synthase enzyme (BAS) issued or derived from a wild-type BAS, in particular from *Rheum palmatum, Wachendorfia thyrsiflora, Rubus ideaus* or *Polygonum cuspidatum,* the recombinant BAS having a T281A substitution as compared to the wild-type BAS from which it is issued or derived.

2. The modified BAS according to claim 1 which has an improved catalytic activity on the transformation of 4-coumaroyl-CoA into 4-hydroxybenzalacetone as compared to the wild-type BAS from which it is issued or derived.

3. The recombinant BAS according to claim 1 or 2, which has at least the same catalytic activity, in particular at least the same catalytic constant k_{cat}, on the transformation of 4-coumaroyl-CoA into 4-hydroxybenzalacetone as compared to a BAS having the amino acid sequence set forth in SEQ ID No. 2.

4. The recombinant BAS according to claim 3, wherein the catalytic activity of the BAS on the transformation of 4-coumaroyl-CoA into 4-hydroxybenzalacetone is determined by measuring by HPLC the total concentration of 4-coumaroyl-CoA and/or 4-hydroxybenzalcetone and/or raspberry ketone and/or 4-(4-hydroxy-3-methoxyphenyl)but-3-en-2-one and/or zingerone, preferably 4-coumaroyl-CoA and/or 4-hydroxybenzalcetone before and after a catalytic reaction of transforming 4-coumaroyl-CoA into 4-hydroxybenzalcetone.

5. The recombinant BAS according to any one of claims 1 to 4, which has at least 90% identity to the wild-type protein from which it is issued, in particular which has at least 90% identity with the BAS having the amino acid sequence set forth in SEQ ID No. 1 or SEQ ID No. 2.

6. The recombinant BAS according to any one of claims 1 to 5, which has the amino acid sequence set forth in SEQ ID No. 2.

7. A nucleic acid molecule encoding the BAS according to any one of claims 1 to 6, comprising or consisting of a gene encoding a modified BAS according to claim 1, in particular a nucleic acid molecule comprising or consisting of the nucleotide sequence set forth in SEQ ID No. 3, or a vector, in particular a plasmid, comprising or consisting of the nucleotide sequence set forth in SEQ ID No. 4.

8. A genetically modified *Pseudomonas putida* strain capable of expressing or expressing a recombinant gene encoding a recombinant benzalacetone synthase (BAS) according to any one of claims 1 to 6, and/or being transformed with a nucleic acid molecule according to claim 7.

9. The genetically modified *Pseudomonas putida* strain according to claim 8, which is capable to produce a 4-hydroxybenzalacetone compound and/or a raspberry ketone compound, in particular from a 4-coumaroyl-CoA compound, or 4-(4-hydroxy-3-methoxyphenyl)but-3-en-2-one and/or zingerone, in particular from ferulic acid.

10. The genetically modified *Pseudomonas putida* strain according to claim 8 or 9, which comprises:
- a gene encoding a tyrosine ammonia lyase; and/or
- a gene encoding a 4-coumarate-ligase, and/or
- a gene encoding a benzalacetone reductase.

11. The genetically modified *Pseudomonas putida* strain according to claim 10, which comprises a gene encoding a tyrosine ammonia lyase; and a gene encoding a 4-coumarate-ligase, and a gene encoding a benzalacetone reductase.

12. Use of a BAS according to any one of claims 1 to 6, for producing a Raspberry ketone compound or a zingerone compound.

13. A method for producing a 4-hydroxybenzalacetone compound and/or a raspberry ketone compound, in particular from a 4-coumaroyl-CoA compound, or 4-(4-hydroxy-3-methoxyphenyl)but-3-en-2-one and/or zingerone, in particular from ferulic acid, comprising a step of using a BAS according to any one of claim 1 to 6, or a genetically modified Pseudomonas putida strain according to any one of claims 8 to 11.

14. A process for producing a 4-hydroxybenzalacetone compound and/or a raspberry ketone compound, in particular from a 4-coumaroyl-CoA compound, or 4-(4-hydroxy-3-methoxyphenyl)but-3-en-2-one and/or zingerone, in particular from ferulic acid, comprising a step of culturing a genetically modified *Pseudomonas putida* strain according to any one of claims 8 to 11, in condition allowing the expression of the recombinant gene encoding the recombinant BAS.
